# EUROPEAN PATENT SPECIFICATION

(11) **EP 2 933 341 B1**
(45) Date of publication and mention of the grant of the patent: **04.10.2017**
(21) Application number: 15163892.1
(22) Date of filing: 16.04.2015
(51) Int. Cl.: C12Q 1/68, C12Q 1/70

(54) **METHODS FOR DETECTING PATHOGEN IN COLDWATER FISH**
VERFAHREN ZUM NACHWEIS VON KRANKHEITSERREGERN IN KALTWASSERFISCH
PROCÉDÉS POUR LA DÉTECTION D'AGENTS PATHOGÈNES DANS DES POISSONS D'EAU FROIDE

(30) Priority: 18.04.2014 US 201461981311 P
(43) Date of publication of application: 21.10.2015
(60) Divisional application: 17160617.1
(73) Proprietor: Schweitzer Biotech Company Ltd., Taipei, 11493 (TW)
(72) Inventor: Hsieh, Wang-Ju, 20251 Keelung City (TW); Kuo, Tsun-Yung, 26341 I-Lan County (TW); Chen, Tai-An, 10687 Taipeh (TW)
(74) Representative: Wittmann, Günther

(56) References cited:
- MITCHELL SUSAN O ET AL: "'Candidatus Branchiomonas cysticola' is a common agent of epitheliocysts in seawater-farmed Atlantic salmon Salmo salar in Norway and Ireland.", DISEASES OF AQUATIC ORGANISMS 13 MAR 2013, vol. 103, no. 1, 13 March 2013 (2013-03-13), pages 35-43, XP009185315, ISSN: 0177-5103
- YUN-LONG TSAI ET AL: "Development of TaqMan Probe-Based Insulated Isothermal PCR (iiPCR) for Sensitive and Specific On-Site Pathogen Detection", PLOS ONE, vol. 7, no. 9, 25 September 2012 (2012-09-25), page e45278, XP055201645, DOI: 10.1371/journal.pone.0045278
- ELENA R. TOENSHOFF ET AL: "A Novel Betaproteobacterial Agent of Gill Epitheliocystis in Seawater Farmed Atlantic Salmon (Salmo salar)", PLOS ONE, vol. 7, no. 3, March 2012 (2012-03), page e32696, XP055201877, DOI: 10.1371/journal.pone.0032696

### SEQUENCE LISTING

<110> UpstartDNA Co., Ltd.
<120> Methods for detecting pathogen in coldwater fish
   <130> P15-0064
   <150> US 61/981,311
   <151> 2014-04-18
   <160> 38
   <170> PatentIn version 3.5
<210> 1
   <211> 25
   <212> DNA
   <213> Artificial Sequence
   <220>
   <223> Forward primer for detecting Candidatus Branchiomonas cysticola.
<400> 1
   gcgaaagtta agctaatacc gcata 25
<210> 2
   <211> 20
   <212> DNA
   <213> Artificial Sequence
   <220>
   <223> Reverse primer for detecting Candidatus Branchiomonas cysticola.
<400> 2
   taatcagcca tcagccgctc 20
<210> 3
   <211> 17
   <212> DNA
   <213> Artificial Sequence
   <220>
   <223> Probe for detecting Candidatus Branchiomonas cysticola.
<400> 3
   caggctttcc tctccca 17
<210> 4
   <211> 19
   <212> DNA
   <213> Artificial Sequence
   <220>
   <223> Forward primer for detecting piscine reovirus.
<400> 4
   tcaacgttat cggctggga 19
<210> 5
   <211> 23
   <212> DNA
   <213> Artificial Sequence
   <220>
   <223> Reverse primer for detecting piscine reovirus.
<400> 5
   gaatccgctg cagatgagta gag 23
<210> 6
   <211> 17
   <212> DNA
   <213> Artificial Sequence
   <220>
   <223> Probe for detecting piscine reovirus.
<400> 6
   ctccaggagt cattgtc 17
<210> 7
   <211> 20
   <212> DNA
   <213> Artificial Sequence
   <220>
   <223> Forward primer for detecting infectious pancreatic necrosis virus.
<400> 7
   tccaaagctc cacactaccg 20
<210> 8
   <211> 21
   <212> DNA
   <213> Artificial Sequence
   <220>
   <223> Reverse primer for detecting infectious pancreatic necrosis virus.
<400> 8
   ctcagacaga ctgccttcga a 21
<210> 9
   <211> 21
   <212> DNA
   <213> Artificial Sequence
   <220>
   <223> Reverse primer for detecting infectious pancreatic necrosis virus.
<400> 9
   tccacctcag acagactgcc t 21
<210> 10
   <211> 16
   <212> DNA
   <213> Artificial Sequence
   <220>
   <223> Probe for detecting infectious pancreatic necrosis virus.
<400> 10
   acgctcaacg ctgcca 16
<210> 11
   <211> 21
   <212> DNA
   <213> Artificial Sequence
   <220>
   <223> Forward primer for detecting salmonid alphavirus.
<400> 11
   caccagtatc tgcctgaacg a 21
<210> 12
   <211> 19
   <212> DNA
   <213> Artificial Sequence
   <220>
   <223> Forward primer for detecting salmonid alphavirus.
<400> 12
   cggacacgtc actggaaac 19
<210> 13
   <211> 22
   <212> DNA
   <213> Artificial Sequence
   <220>
   <223> Forward primer for detecting salmonid alphavirus.
<400> 13
   gaaaccacca gtatctgcct ga 22
<210> 14
   <211> 20
   <212> DNA
   <213> Artificial Sequence
   <220>
   <223> Reverse primer for detecting salmonid alphavirus.
<400> 14
   cacgtcctgg tacactgcaa 20
<210> 15
   <211> 22
   <212> DNA
   <213> Artificial Sequence
   <220>
   <223> Reverse primer for detecting salmonid alphavirus.
<400> 15
   tcctggtaca ctgcaatatc gg 22
<210> 16
   <211> 21
   <212> DNA
   <213> Artificial Sequence
   <220>
   <223> Reverse primer for detecting salmonid alphavirus.
<400> 16
   cctggtacac tgcaatatcg g 21
<210> 17
   <211> 19
   <212> DNA
   <213> Artificial Sequence
   <220>
   <223> Probe for detecting salmonid alphavirus.
<400> 17
   cgtgagttgt aaagtaaag 19
<210> 18
   <211> 20
   <212> DNA
   <213> Artificial Sequence
   <220>
   <223> Forward primer for detecting infectious salmon anemia virus.
<400> 18
   ccaattggtg aacggaatga 20
   <210> 19
   <211> 22
   <212> DNA
   <213> Artificial Sequence
   <220>
   <223> Forward primer for detecting infectious salmon anemia virus.
<400> 19
   ccagaaggga aaatggtgaa ag 22
<210> 20
   <211> 20
   <212> DNA
   <213> Artificial Sequence
   <220>
   <223> Reverse primer for detecting infectious salmon anemia virus.
<400> 20
   tcatcagtgt cgccatgctt 20
<210> 21
   <211> 20
   <212> DNA
   <213> Artificial Sequence
   <220>
   <223> Reverse primer for detecting infectious salmon anemia virus.
<400> 21
   ctcatcagtg tcgccatgct 20
<210> 22
   <211> 22
   <212> DNA
   <213> Artificial Sequence
   <220>
   <223> Probe for detecting infectious salmon anemia virus.
<400> 22
   cgacgatgac tctctactgt gt 22
<210> 23
   <211> 19
   <212> DNA
   <213> Artificial Sequence
   <220>
   <223> Forward primer for detecting Candidatus Branchiomonas cysticola.
<400> 23
   ttgacatgtg cggaagctg 19
<210> 24
   <211> 20
   <212> DNA
   <213> Artificial Sequence
   <220>
   <223> Reverse primer for detecting Candidatus Branchiomonas cysticola.
<400> 24
   ccaacatctc acgacacgag 20
<210> 25
   <211> 22
   <212> DNA
   <213> Artificial Sequence
   <220>
   <223> Forward primer for detecting Candidatus Branchiomonas cysticola.
<400> 25
   gacatgtgcg gaagctgtaa ga 22
<210> 26
   <211> 17
   <212> DNA
   <213> Artificial Sequence
   <220>
   <223> Reverse primer for detecting Candidatus Branchiomonas cysticola.
<400> 26
   acgacagcca tgcagca 17
<210> 27
   <211> 18
   <212> DNA
   <213> Artificial Sequence
   <220>
   <223> Forward primer for detecting Candidatus Branchiomonas cysticola.
<400> 27
   tgacatgtgc ggaagctg 18
<210> 28
   <211> 19
   <212> DNA
   <213> Artificial Sequence
   <220>
   <223> Reverse primer for detecting Candidatus Branchiomonas cysticola.
<400> 28
   cccaacatct cacgacacg 19
<210> 29
   <211> 15
   <212> DNA
   <213> Artificial Sequence
   <220>
   <223> Probe for detecting Candidatus Branchiomonas cysticola.
<400> 29
   cggcgtgcct gagaa 15
<210> 30
   <211> 17
   <212> DNA
   <213> Artificial Sequence
   <220>
   <223> Forward primer for detecting piscine reovirus.
<400> 30
   atcggctggg agcaatg 17
<210> 31
   <211> 20
   <212> DNA
   <213> Artificial Sequence
   <220>
   <223> Reverse primer for detecting piscine reovirus.
<400> 31
   tctactccgt tgaatccgct 20
<210> 32
   <211> 17
   <212> DNA
   <213> Artificial Sequence
   <220>
   <223> Forward primer for detecting piscine reovirus.
<400> 32
   tgggagcaat gggatgc 17
<210> 33
   <211> 22
   <212> DNA
   <213> Artificial Sequence
   <220>
   <223> Forward primer for detecting piscine reovirus.
<400> 33
   tctctggtct caacgttatc gg 22
<210> 34
   <211> 17
   <212> DNA
   <213> Artificial Sequence
   <220>
   <223> Reverse primer for detecting piscine reovirus.
<400> 34
   ccgttgaatc cgctgca 17
<210> 35
   <211> 23
   <212> DNA
   <213> Artificial Sequence
   <220>
   <223> Forward primer for detecting piscine reovirus.
<400> 35
   gggttctctg gtctcaacgt tat 23
<210> 36
   <211> 19
   <212> DNA
   <213> Artificial Sequence
   <220>
   <223> Reverse primer for detecting piscine reovirus.
<400> 36
   accggcgaca atgactcct 19
<210> 37
   <211> 19
   <212> DNA
   <213> Artificial Sequence
   <220>
   <223> Probe for detecting piscine reovirus.
<400> 37
   caatgggatg ctaacactc 19
<210> 38
   <211> 21
   <212> DNA
   <213> Artificial Sequence
   <220>
   <223> Probe for detecting infectious salmon anemia virus.
<400> 38
   cgatgactct ctactgtgtg a 21

## Description

### BACKGROUND OF THE INVENTION

### 1. FIELD OF THE INVENTION

The invention relates to the field of methods and pairs of oligonucleotides for detecting pathogens in coldwater fish.

### 2. DESCRIPTION OF THE PRIOR ART

Economic value of coldwater fish reached into the USD billions with Atlantic salmon being the largest single specie cultured with more than 1.5 million metric tons in 2013. The cold water diseases can cause mortalities up to more than 90% but are more usually found in the 10-30% range, still a huge negative economical factor for the aquaculture, and with a negative value of many hundred million USD a year. Diseases may occur at the early stage in the production cycle as well as the end, where the relative economic consequences are the largest.

To be able to diagnose and take necessary steps to avoid huge mortalities is a key and the invention shall be able to provide such diagnosis quickly and decentralized, as the coldwater aquaculture is normally spread out in remote costal areas.

MITCHELL SUSAN ET AL: 'Candidatus Branchiomonas cysticola' is a common agent of epitheliocysts in seawater-farmed Atlantic salmon Salmo salar in Norway and Ireland, DISEASES OF AQUATIC ORGANISMS 13 MAR 2013, vol. 103. no. 1, 13 March 2013, pages 35-43 relates to detection of Candidatus Branchiomonas cysticola in seawater-farmed fish.

### SUMMARY OF THE INVENTION

The invention is defined by the scope of the appended claims. In one aspect, the disclosure relates to a method for detecting a pathogen in coldwater fish, comprising providing a sample potentially containing one or more nucleotide sequences of a pathogen in a coldwater fish, providing an oligonucleotide primer pair defining the 5' ends of two complementary strands of a double stranded target sequence on the one or more nucleotide sequences of the pathogen, providing a polymerase, blending the sample, the oligonucleotide primer pair, the polymerase, deoxyadenosine triphosphates (dATPs), deoxycytidine triphosphates (dCTPs), deoxyguanosine triphosphates (dGTPs), and deoxythymidine triphosphates (dTTPs) in a container to form a polymerase chain reaction (PCR) mixture, subjecting the PCR mixture to insulated isothermal polymerase chain reaction (iiPCR) by heating the bottom of the container at a fixed temperature to form a PCR product, and detecting the PCR product to identify the double stranded target sequence. The PCR mixture further comprises an oligonucleotide probe having a sequence complementary to a segment of the double stranded target sequence, a fluorescer molecule attached to a first location on the oligonucleotide probe, and a quencher molecule attached to a second location on the oligonucleotide probe such that the quencher molecule substantially quenches the fluorescer molecule whenever the oligonucleotide probe is not hybridized to the segment of the double stranded target sequence and such that the fluorescer molecule is substantially unquenched whenever the oligonucleotide probe is hybridized to the segment of the double stranded target sequence.

In some embodiments, the pathogen is *Candidatus Branchiomonas cysticola,* and the oligonucleotide primer pair comprises a first primer having the sequence of SEQ ID NO: 1 and a second primer having the sequence of SEQ ID NO: 2. The PCR mixture further comprises an oligonucleotide probe, which is a 13- to 25-base pair (bp) oligonucleotide between the 136th to 225th nucleotides of 16S ribosomal RNA gene of *Candidatus Branchiomonas cysticola* (GenBank accession No. JQ723599). The oligonucleotide probe has the sequence of SEQ ID NO: 3.

In some embodiments, the pathogen is *Candidatus Branchiomonas cysticola,* and the oligonucleotide primer pair comprises a first primer having a sequence selected from the group consisting of SEQ ID NOs: 23, 25, or 27, and a second primer having a sequence selected from the group consisting of SEQ ID NOs: 24, 26, or 28. The PCR mixture further comprises an oligonucleotide probe, which is a 13- to 25-bp oligonucleotide between the 968th to 1068th nucleotides of 16S ribosomal RNA gene of *Candidatus Branchiomonas cysticola* (GenBank accession No. JQ723599). The oligonucleotide probe has the sequence of SEQ ID NO: 29. Further disclosed are embodiments, wherein the pathogen is piscine reovirus (PRV), and the oligonucleotide primer pair comprises a first primer having a sequence of SEQ ID NOs: 4, 30, 32, 33, or 35, and a second primer having a sequence of SEQ ID NOs: 5, 31, 34, or 36. In some embodiments, the PCR mixture further comprises an oligonucleotide probe, which is a 13- to 25-bp oligonucleotide between the 3178th to 3287th nucleotides of segment L1 gene of PRV (GenBank accession No. GU994013). In some preferable embodiments, the oligonucleotide probe has a sequence of SEQ ID NOs: 6 or 37. Further disclosed are embodiments, wherein the pathogen is infectious pancreatic necrosis virus (IPNV), and the oligonucleotide primer pair comprises a first primer having a sequence of SEQ ID NO: 7 and a second primer having a sequence of SEQ ID NOs: 8 or 9. In some embodiments, the PCR mixture further comprises an oligonucleotide, which is a 13- to 25-bp oligonucleotide between the 432nd to 519th nucleotides of segment A gene of IPNV (GenBank accession No. AY379740). In some preferable embodiments, the oligonucleotide probe has a sequence of SEQ ID NO: 10. Further disclosed are embodiments, wherein the pathogen is salmonid alphavirus (SAV), and the oligonucleotide primer pair comprises a first primer having a sequence selected from a group consisting of SEQ ID NOs: 11, 12, and 13, and a second primer having a sequence selected from a group consisting of SEQ ID NOs: 14, 15, and 16. In some embodiments, the PCR mixture further comprises an oligonucleotide probe, which is a 13- to 25-bp oligonucleotide between the 446th to 534th nucleotides of SAV complete genome (GenBank accession No. KC122926). In some preferable embodiments, the oligonucleotide probe has a sequence of SEQ ID NO: 17. Further disclosed are embodiments, wherein the pathogen is infectious salmon anemia virus (ISAV), and the oligonucleotide primer pair comprises a first primer having a sequence of SEQ ID NO: 18 or SEQ ID NO: 19 and a second primer having a sequence of SEQ ID NO: 20 or SEQ ID NO: 21. In some embodiments, the PCR mixture further comprises an oligonucleotide probe, which is a 13- to 25-bp oligonucleotide between the 178th to 305th nucleotides of non-structural protein and matrix protein genes of ISAV (GenBank accession No. DQ785286). In some preferable embodiments, the oligonucleotide probe has a sequence of SEQ ID NOs: 22 or 38.

In one aspect, the disclosure relates to a set of oligonucleotides for detecting pathogens in coldwater fish.

In some embodiments, the pathogen is *Candidatus Branchiomonas cysticola,* and the set of oligonucleotide comprises a first primer having a sequence of SEQ ID NO: 1 and a second primer having a sequence of SEQ ID NO: 2. The set of oligonucleotides further comprises an oligonucleotide probe having a 13- to 25-bp oligonucleotide between the 136th to 225th nucleotides of 16S ribosomal RNA gene of *Candidatus Branchiomonas cysticola* (GenBank accession No. JQ723599), a fluorescer molecule attached to a first location on the oligonucleotide probe, and a quencher molecule attached to a second location on the oligonucleotide probe. The oligonucleotide probe has the sequence of SEQ ID NO: 3.

In some embodiments, the pathogen is *Candidatus Branchiomonas cysticola,* and the set of oligonucleotide comprises a first primer having a sequence selected from the group consisting of SEQ ID NOs: 23, 25, or 27, and a second primer having a sequence selected from the group consisting of SEQ ID NOs: 24, 26, or 28. The set of oligonucleotides further comprises an oligonucleotide probe having a 13- to 25-bp oligonucleotide between the 968th to 1068th nucleotides of 16S ribosomal RNA gene of *Candidatus Branchiomonas cysticola* (GenBank accession No. JQ723599), a fluorescer molecule attached to a first location on the oligonucleotide probe, and a quencher molecule attached to a second location on the oligonucleotide probe. The oligonucleotide probe has the sequence of SEQ ID NO: 29. Further disclosed are embodiments, wherein the pathogen is piscine reovirus (PRV), and the pair of oligonucleotides comprises a first primer having a sequence of SEQ ID NOs: 4, 30, 32, 33, or 35, and a second primer having a sequence of SEQ ID NOs: 5, 31, 34, or 36. In some embodiments, the pair of oligonucleotides further comprises an oligonucleotide probe having a 13- to 25-bp oligonucleotide between the 3178th to 3287th nucleotides of segment L1 gene of PRV (GenBank accession No. GU994013), a fluorescer molecule attached to a first location on the oligonucleotide probe, and a quencher molecule attached to a second location on the oligonucleotide probe. In some preferable embodiments, the oligonucleotide probe has a sequence of SEQ ID NOs: 6 or 37. Further disclosed are embodiments, wherein the pathogen is infectious pancreatic necrosis virus (IPNV), and the pair of oligonucleotides comprises a first primer having a sequence of SEQ ID NO: 7 and a second primer having a sequence of SEQ ID NOs: 8 or 9. In some embodiments, the pair of oligonucleotides further comprises an oligonucleotide probe having a 13- to 25-bp oligonucleotide between the 432nd to 519th nucleotides of segment A gene of IPNV (GenBank accession No. AY379740), a fluorescer molecule attached to a first location on the oligonucleotide probe, and a quencher molecule attached to a second location on the oligonucleotide probe. In some preferable embodiments, the oligonucleotide probe has a sequence of SEQ ID NO: 10. Further disclosed are embodiments, wherein the pathogen is salmonid alphavirus (SAV), and the pair of oligonucleotides comprises a first primer having a sequence selected from a group consisting of SEQ ID NOs: 11, 12, and 13, and a second primer having a sequence selected from a group consisting of SEQ ID NOs: 14, 15, and 16. In some embodiments, the pair of oligonucleotides further comprises an oligonucleotide probe having a 13- to 25-bp oligonucleotide between the 446th to 534th nucleotides of SAV complete genome (GenBank accession No. KC 122926), a fluorescer molecule attached to a first location on the oligonucleotide probe, and a quencher molecule attached to a second location on the oligonucleotide probe. In some preferable embodiments, the oligonucleotide probe has a sequence of SEQ ID NO: 17. Further disclosed are embodiments, wherein the pathogen is infectious salmon anemia virus (ISAV), and the pair of oligonucleotides comprises a first primer having a sequence of SEQ ID NOs: 18 or 19 and a second primer having a sequence of SEQ ID NOs: 20 or 21. In some embodiments, the pair of oligonucleotides further comprises an oligonucleotide probe having a 13- to 25-bp oligonucleotide between the 178th to 305th nucleotides of non-structural protein and matrix protein genes of ISAV (GenBank accession No. DQ785286), a fluorescer molecule attached to a first location on the oligonucleotide probe, and a quencher molecule attached to a second location on the oligonucleotide probe. In some preferable embodiments, the oligonucleotide probe has a sequence of SEQ ID NOs: 22 or 38.

### BRIEF DESCRIPTION OF THE DRAWINGS

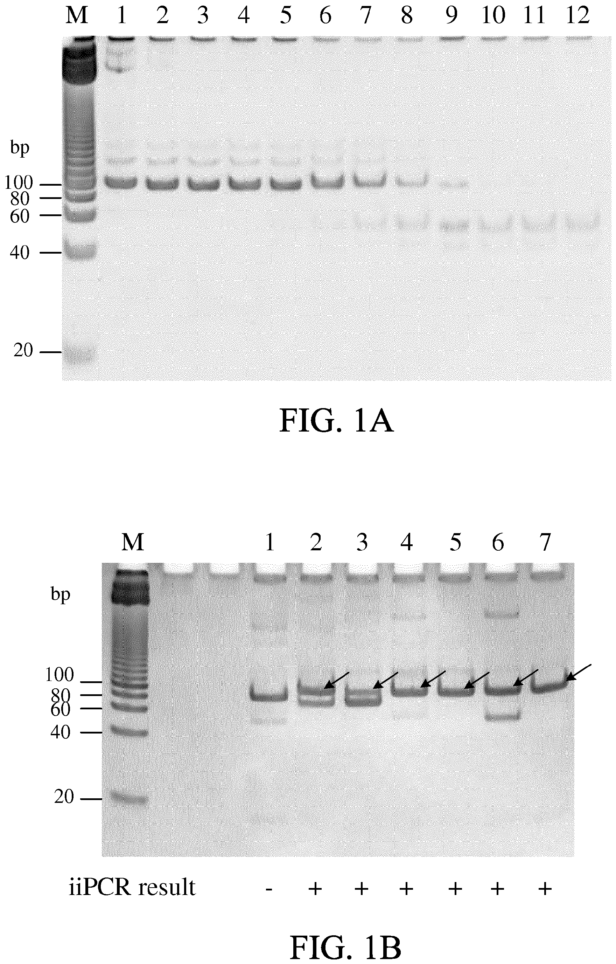

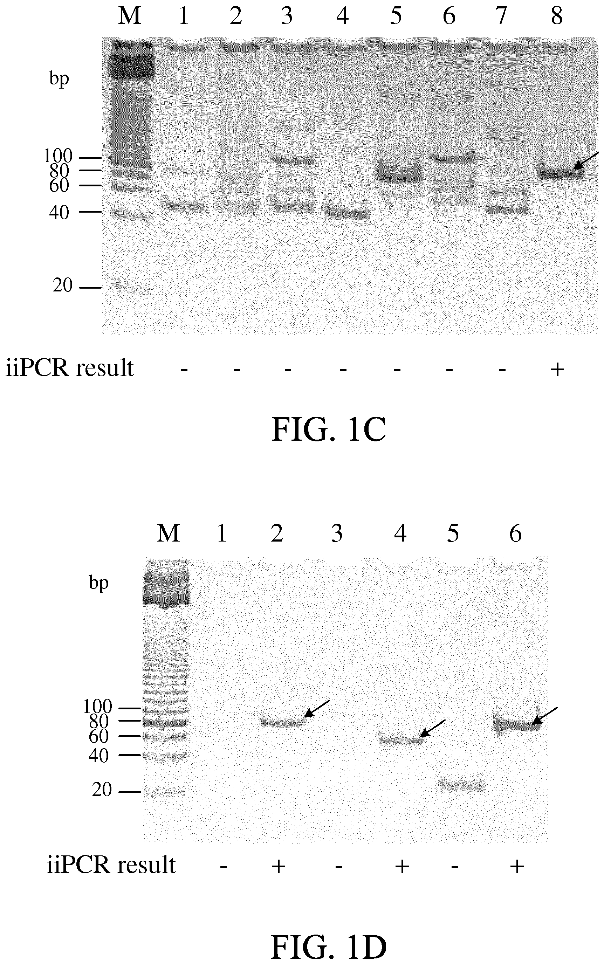

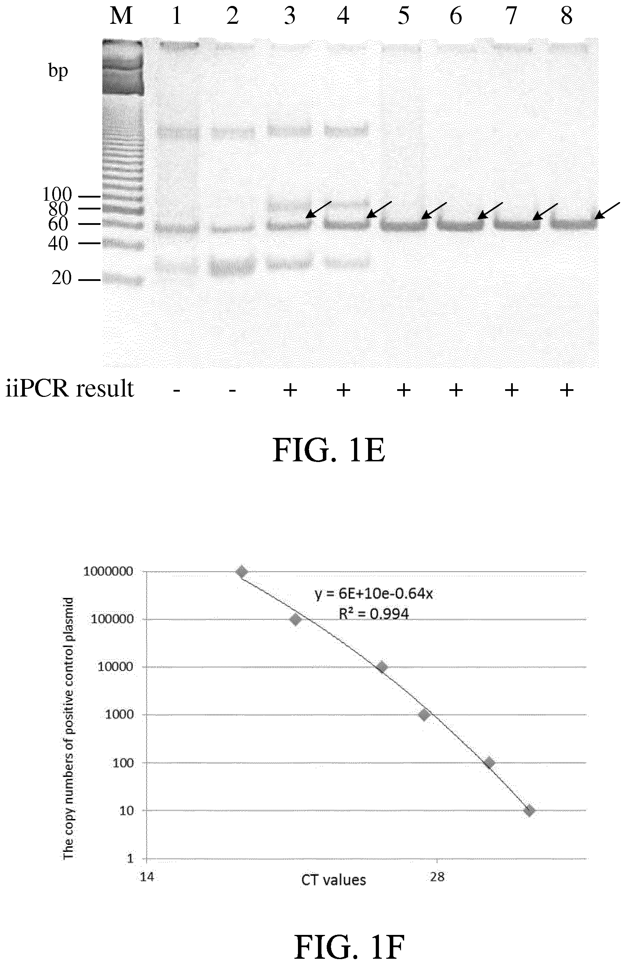

**FIG. 1A** shows the results of conventional PCR test of *Candidatus Branchiomonas cysticola* with primers BCF1 and BCR1; lanes 1 to 11: results of conventional PCR performed with 10¹⁰, 10⁹, 10⁸, 10⁷, 10⁶, 10⁵, 10⁴, 10³, 10², 10¹, 10⁰ copies of pUC57-BC plasmid respectively; lane 12: results of conventional PCR performed without DNA template (negative control). **FIG**. **1B** shows the results of iiPCR test of primers BCF1 and BCR1 and probe BCP1; lane 1: result of iiPCR performed without DNA template (negative control); lanes 2 to 7: results of iiPCR performed with 10¹, 10², 10³, 10⁴, 10⁵, 10⁶ copies of pUC57-BC plasmid respectively. **FIG. 1C** shows the results of iiPCR test for specificity of primers BCF1 and BCR1 and probe BCP21; lane 1: result of iiPCR performed without DNA template (negative control); lanes 2 to 8: results of iiPCR performed with DNA/RNA template of salmon gill, SAV, IPNV, ISAV, NNV, PRV, and pUC57-BC plasmid. **FIG**. **ID** shows the results of iiPCR test for different primers and probe BCP2; lanes 1 and 2: results of iiPCR performed with primers BCF2 and BCR2 and probe BCP2 without DNA template (lane 1, negative control) and with DNA template (lane 2); lanes 3 and 4: results of iiPCR performed with primers BCF3 and BCR3 and probe BCP2 without DNA template (lane 3, negative control) and with DNA template (lane 4); lanes 5 and 6: results of iiPCR performed with primers BCF4 and BCR4 and probe BCP2 without DNA template (lane 5, negative control) and with DNA template (lane 6). **FIG. 1E** shows the results of iiPCR test for sensitivity of primers BCF3, BCR3, and probe BCP2; lane 1: result of iiPCR performed without DNA template (negative control); lanes 2 to 8: results of iiPCR performed with 10⁰, 10¹, 10², 10¹, 10⁴, 10¹, 10⁶ copies of pUC57-BC plasmid respectively. **FIG**. **1F** shows the results of real-time PCR test of *Candidatus Branchiomonas cysticola,* pUC57-BC plasmid, with primers BCF3 and BCR3. "M" represents molecular weight marker; arrows indicate the target PCR products; "+" indicates fluorescent signal was detected in the sample, and "-" indicates fluorescent signal was not detected in the sample.

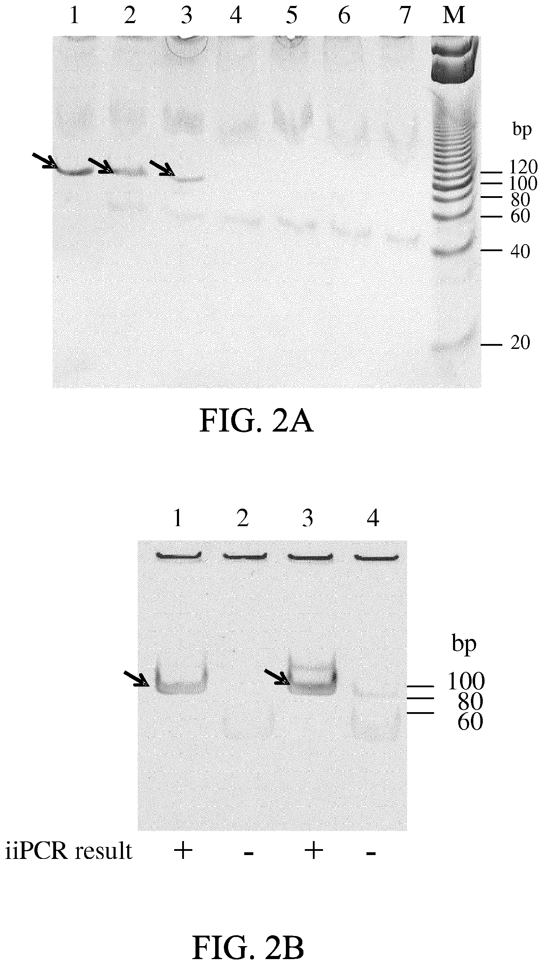

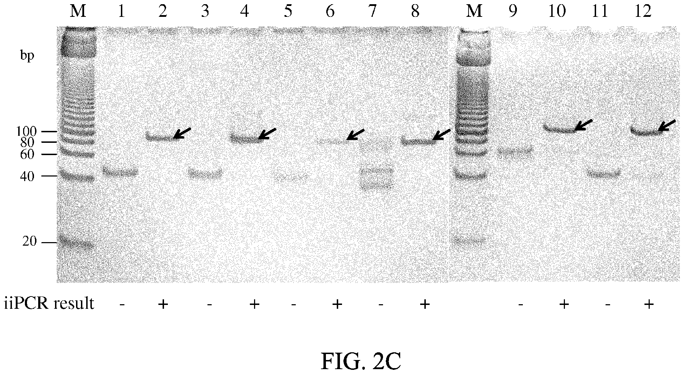

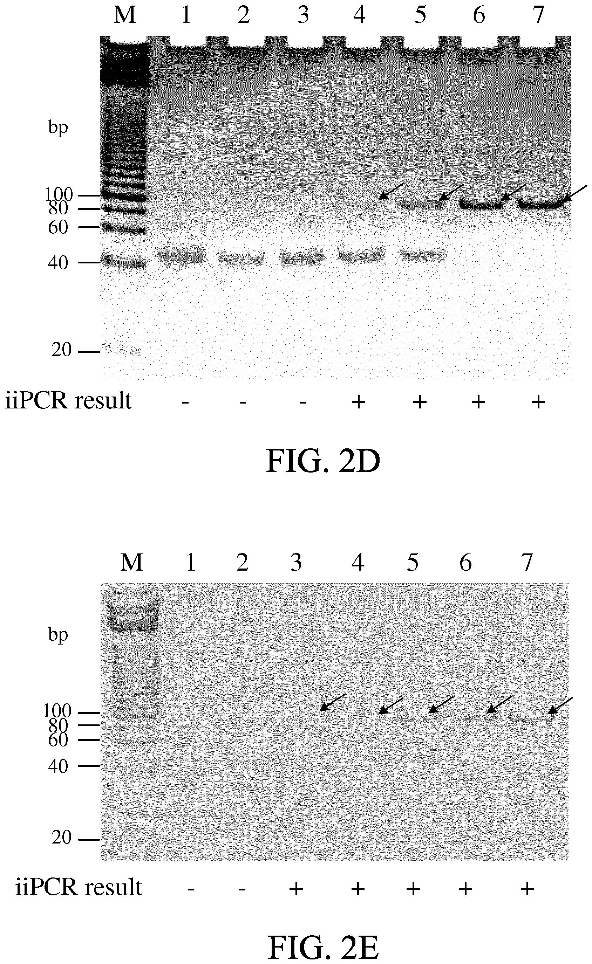

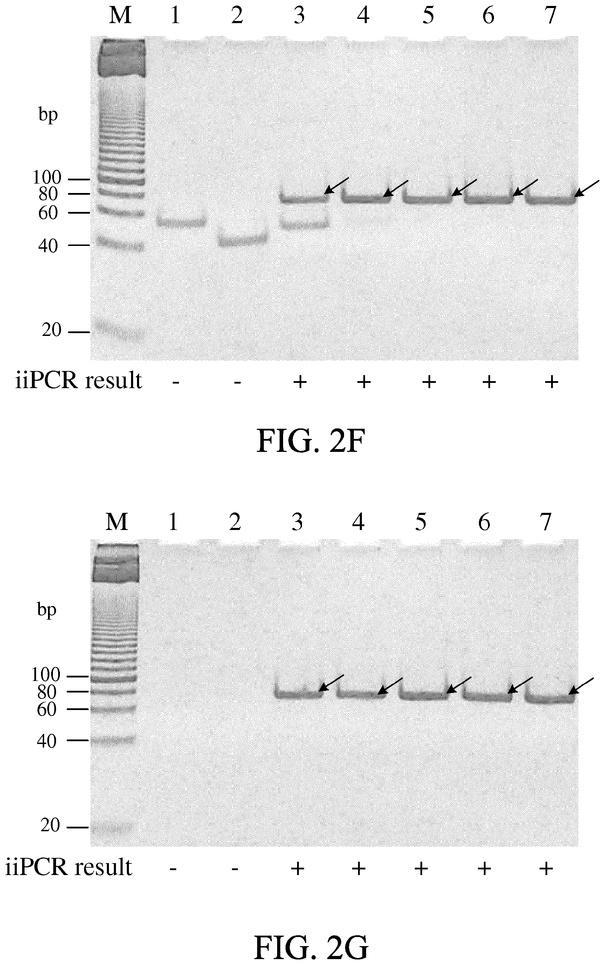

**FIG. 2A** shows the results of conventional PCR test of piscine reovirus (PRV) with primers PRVF 1 and PRVR1; lanes 1 to 6: results of conventional PCR performed with 10⁶, 10⁵, 10⁴, 10³, 10², 10¹ copies of pUC57-PRV plasmid respectively; lane 7: results of conventional PCR performed without DNA template (negative control). **FIG. 2B** shows the results of iiPCR test of primers PRVF 1 and PRVR1 and probe PRV1; lanes 1 and 3: results of iiPCR performed with pUC57-PRV; lanes 2 and 4: results of iiPCR performed without DNA template (negative controls). **FIG. 2C** shows the results of iiPCR test of different primers and probe PRVP1; lanes 1 and 3: results of iiPCR performed with primers PRVF2, PRVR2 and probe PRVP1, and without DNA template (negative controls); lanes 2 and 4: results of iiPCR performed with primers PRVF2, PRVR2 and probe PRVP1, and with pUC57-PRV; lanes 5 and 7: results of iiPCR performed with primers PRVF3, PRVR2 and probe PRV1, and without DNA template (negative controls); lanes 6 and 8: results of iiPCR performed with primers PRVF3, PRVR2 and probe PRVP1, and with pUC57-PRV; lanes 9 and 11: results of iiPCR performed with primers PRVF4, PRVR4 and probe PRVP1, and without DNA template (negative controls); lanes 10 and 12: results of iiPCR performed with primers PRVF4, PRVR4 and probe PRVP1, and with pUC57-PRV. **FIG. 2D** shows the results of iiPCR test for sensitivity of primers PRVF2, PRVR2, and probe PRVP1; lane 1: result of iiPCR performed without DNA template (negative control); lanes 2 to 7: results of iiPCR performed with 10¹, 10², 10³, 10⁴, 10⁵, 10⁶ copies of in vitro transcriptional RNA of PRV respectively. **FIG. 2E** shows the results of iiPCR test for sensitivity of primers PRVF3, PRVR2, and probe PRVP1; lane 1: result of iiPCR performed without DNA template (negative control); lanes 2 to 7: results of iiPCR performed with 10¹, 10², 10³, 10⁴, 10⁵, 10⁶ copies of in vitro transcriptional RNA of PRV respectively. **FIG. 2F** shows the results of iiPCR test for sensitivity of primers PRVF5, PRVR5, and probe PRVP2; lane 1: result of iiPCR performed without DNA template (negative control); lanes 2 to 7: results of iiPCR performed with 10¹, 10², 10¹, 10⁴, 10⁵, 10⁶ copies of pUC57-PRV plasmid respectively. **FIG**. **2G** shows the results of iiPCR test for sensitivity of primers PRVF5, PRVR5, and probe PRVP2; lane 1: result of iiPCR performed without DNA template (negative control); lanes 2 to 7: results of iiPCR performed with 10¹, 10², 10³, 10⁴, 10⁵, 10⁶ copies of in vitro transcriptional RNA of PRV respectively. "M" represents molecular weight marker; arrows indicate the target PCR products; "+" indicates fluorescent signal was detected in the sample, and "-" indicates fluorescent signal was not detected in the sample.

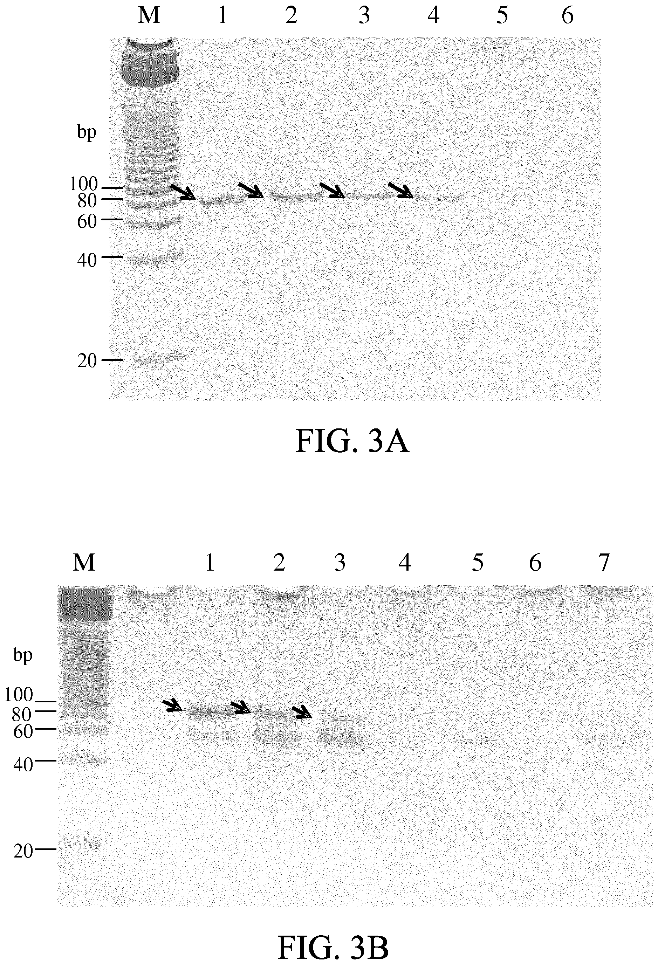

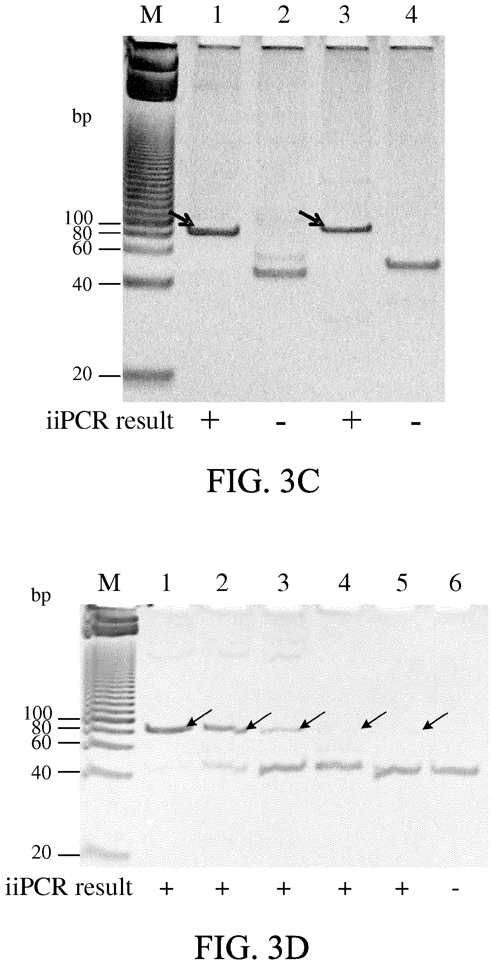

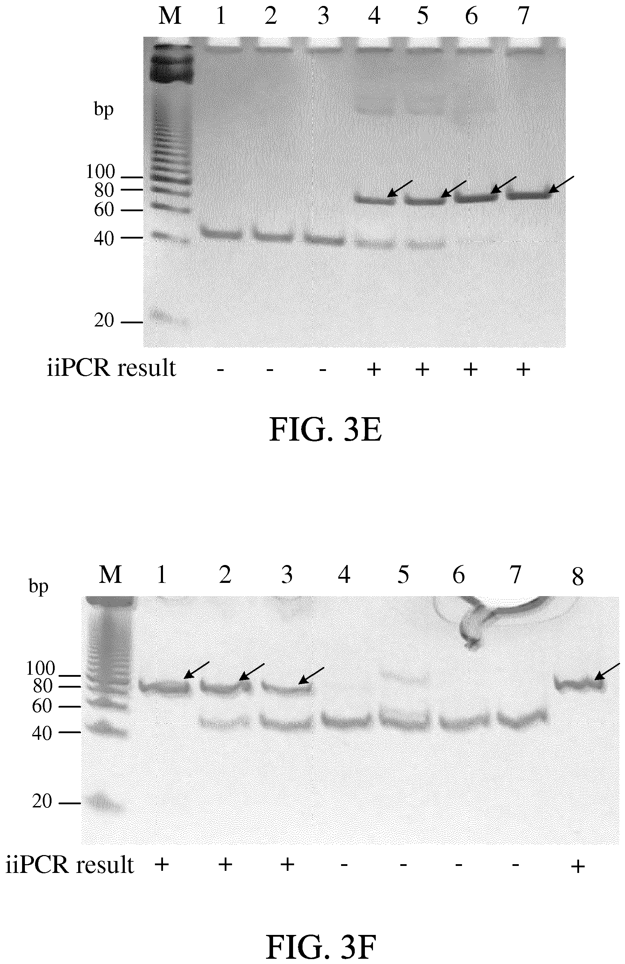

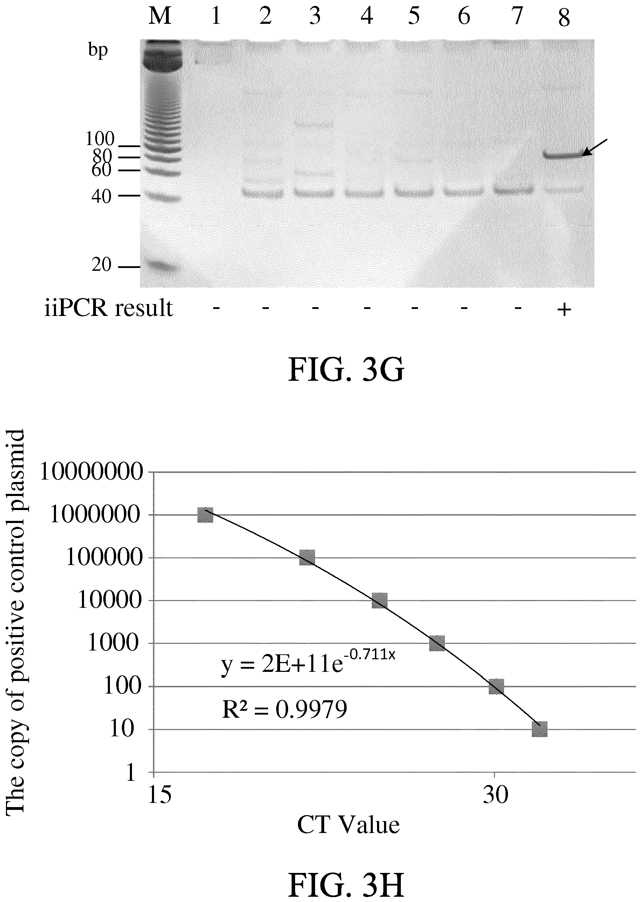

**FIG. 3A** shows the results of conventional PCR test of infectious pancreatic necrosis virus (IPNV) with primers IPNVF1 and IPNVR1; lanes 1 to 5: results of conventional PCR performed with 10⁵, 10⁴, 10³, 10², 10¹ copies of pTA-IPNV plasmid respectively; lane 6: results of conventional PCR performed without DNA template (negative control). **FIG. 3B** shows the results of RT-PCR (reverse transcription PCR) test of IPNV with primers IPNVF 1 and IPNVR1; lanes 1 to 6: results of RT-PCR performed with 10², 10¹, 10⁴, 10⁵, 10⁶, 10⁷ -fold diluted total RNA respectively; lane 7: results of RT-PCR performed without RNA sample (negative control). **FIG. 3C** shows the results of iiPCR test of IPNV with different primers; lane 1: result of iiPCR performed with pTA-IPNV and primers IPNVF1 and IPNVR1 and probe IPNVP1; lane 2: result of iiPCR performed with primers IPNVF1 and IPNVR1 and probe IPNVP1 but without DNA template (negative controls); lane 3: result of iiPCR performed with pTA-IPNV and IPNVF1 and IPNVR2 and probe IPNVP1; lane 4: result of iiPCR performed with IPNVF1 and IPNVR2 and probe IPNVP1 but without DNA template (negative controls). **FIG. 3D** shows the results of iiPCR test for sensitivity of primers IPNVF1, IPNVR1, and probe IPNVP1 with pTA-IPNV plasmid; lanes 1 to 5: results of iiPCR performed with 10⁵, 10⁴, 10³, 10², 10¹ copies of pTA-IPNV plasmid respectively; lane 6: result of iiPCR performed without DNA template (negative control). **FIG. 3E** shows the results of iiPCR test for sensitivity of primers IPNVF1, IPNVR1, and probe IPNVP1 with in vitro transcriptional RNA of IPNV; lane 1: result of iiPCR performed without DNA template (negative control); lanes 2 to 7: results of iiPCR performed with 10¹, 10², 10¹, 10⁴, 10⁵, 10⁶ copies of in vitro transcriptional RNA of IPNV respectively. **FIG. 3F** shows the results of iiPCR test for sensitivity of primers IPNVF1, IPNVR1, and probe IPNVP1 with serially diluted RNAofIPNV; lanes 1 to 6: results of iiPCR performed with stock (10⁰), 10¹, 10⁴, 10⁵, 10⁶, 10⁷ -fold diluted total RNA of IPNV respectively; lane 7: result of iiPCR performed without RNA template (negative control); lane 8: result of iiPCR performed with DNA template (positive control). **FIG. 3G** shows the results of iiPCR test for specificity of primers IPNVF1, IPNVR1, and probe IPNVP1; lane 6: result of iiPCR performed without RNA template (negative control); lanes 1 to 5, and 7: results of iiPCR performed with DNA/RNA template of ISAV, SAV, NNV, PRV, salmon muscle tissue, and pTA-IPNV plasmid respectively. **FIG. 3H** shows the results of real-time PCR test of infectious pancreatic necrosis virus (IPNV), pTA-IPNV plasmid, with primers IPNVF 1, IPNVR1. "M" represents molecular weight marker; arrows indicate the target PCR products; "+" indicates fluorescent signal was detected in the sample, and "-" indicates fluorescent signal was not detected in the sample.

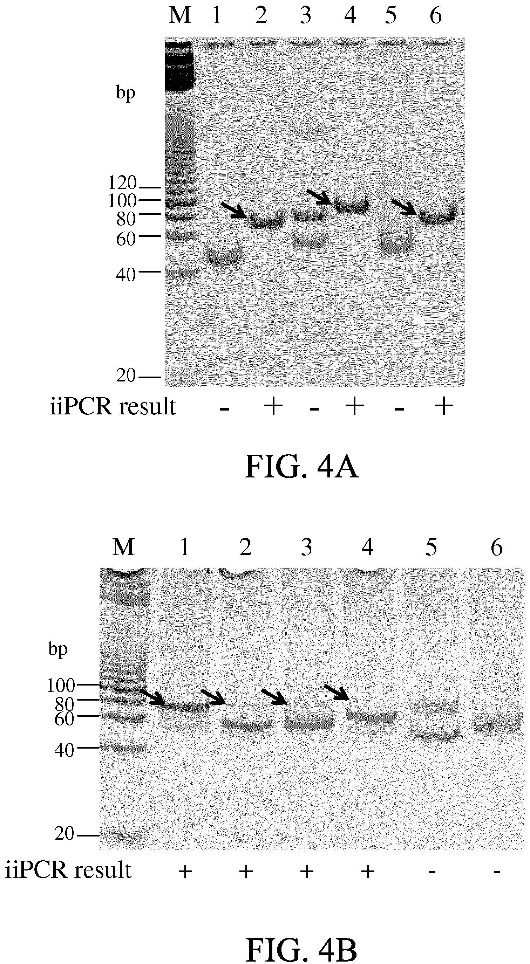

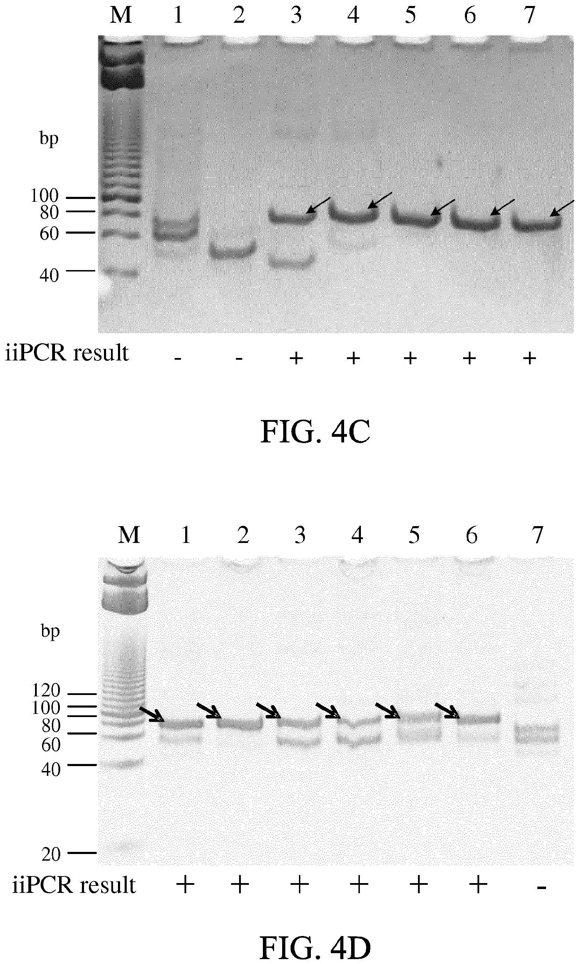

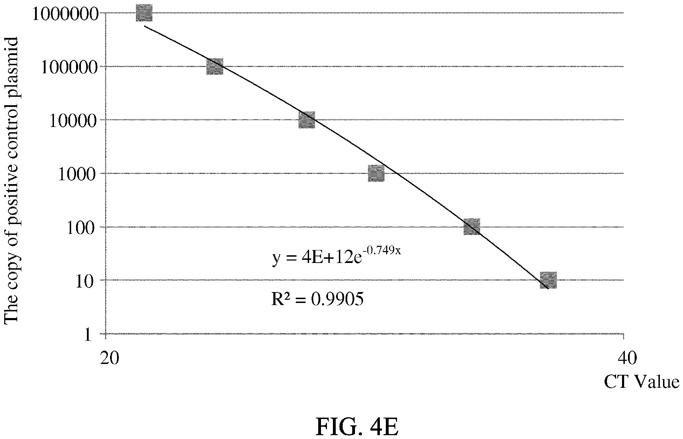

**FIG. 4A** shows the results of iiPCR test of salmonid alphavirus (SAV) with different primers; lanes 1,3, and 5: result of iiPCR performed without pTA-SAV but with primers SAVF1 and SAVR1, primers SAVF2 and SAVR2, and primers SAVF3 and SAVR3 (negative controls); lanes 2, 4, and 6: result of iiPCR performed with pTA-SAV and primers SAVF1 and SAVR1, primers SAVF2 and SAVR2, primers SAVF3 and SAVR3 respectively. **FIG. 4B** shows the results of iiPCR test for sensitivity of primers SAVF1 and SAVR1, and probe SAVP1 with pTA-SAV plasmid; lanes 1 to 5: results of iiPCR performed with 10⁵, 10⁴, 10³, 10², 10¹ copies of pTA-SAV plasmid respectively; lane 6: result of iiPCR performed without DNA template (negative control). **FIG. 4C** shows the results of iiPCR test for sensitivity of primers SAVF1 and SAVR1, and probe SAVP1 with in vitro transcriptional RNA of SAV; lane 1: result of iiPCR performed without RNA template (negative control); lanes 2 to 7: results of iiPCR performed with 10¹, 10², 10³, 10⁴, 10⁵, 10⁶ copies of in vitro transcriptional RNA of SAV respectively. **FIG. 4D** shows the results of iiPCR test for sensitivity of primers SAVF1 and SAVR1, and probe SAVP1 with serially diluted RNA of SAV; lane 1: result of iiPCR performed with DNA template (positive control); lanes 2 to 6: results of iiPCR performed with 10¹, 10³, 10⁴, 10⁵, 10⁶ -fold diluted total RNA of SAV respectively; lane 7: result of iiPCR performed without RNA template (negative control). **FIG. 4E** shows the results of real-time PCR test of SAV, pTA-SAV plasmid, with primers SAVF1 and SAVR1. "M" represents molecular weight marker; arrows indicate the target PCR products; "+" indicates fluorescent signal was detected in the sample, and "-" indicates fluorescent signal was not detected in the sample.

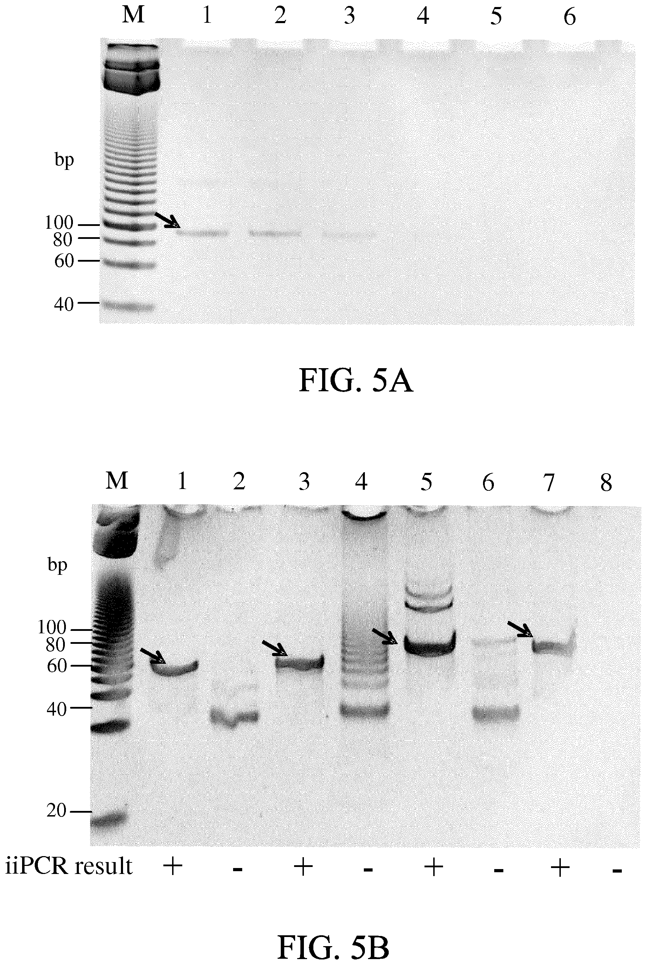

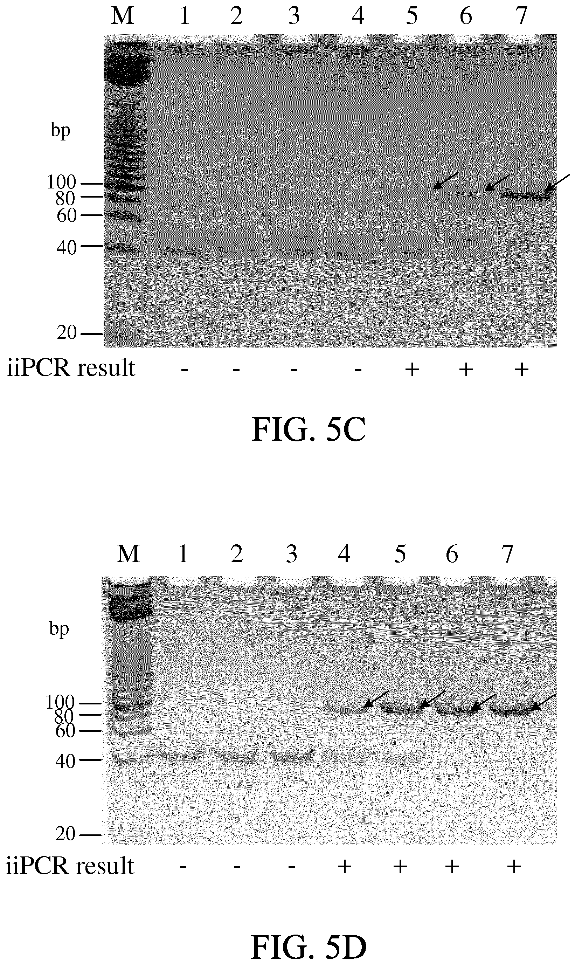

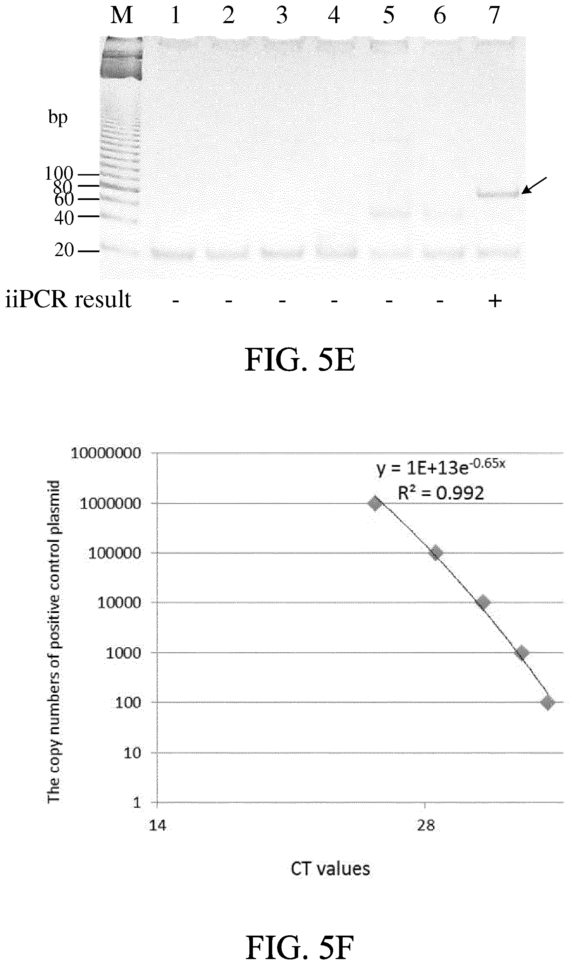

**FIG. 5A** shows the results of conventional PCR test of infectious salmonanemia virus (ISAV) with primers ISAVF1 and ISAVR1; lanes 1 to 5: results of conventional PCR performed with 10⁵, 10⁴, 10³, 10², 10¹ copies of pTA-ISAV plasmid respectively; lane 6: results of conventional PCR performed without DNA template (negative control). **FIG. 5B** shows the results of iiPCR test of ISAV with different primers; lanes 1, 3, 5, and 7: results of iiPCR performed with pTA-ISAV plasmid and primers ISAVF1 1 and ISAVR1, primers ISAVF1 and ISAVR2, primers ISAVF2 and ISAVR2, primers ISAVF2 and ISAVR1 respectively; lanes 2, 4, 6, and 8: results of iiPCR performed without DNA but with primers ISAVF1 and ISAVR1, primers ISAVF1 and ISAVR2, primers ISAVF2 and ISAVR2, primers ISAVF2 and ISAVR1 respectively (negative controls). **FIG. 5C** shows the results of iiPCR test for sensitivity of primers ISAVF1 and ISAVR1, and probe ISAVP2 with pTA-ISAV plasmid; lane 1: result of iiPCR performed without DNA template (negative control); lanes 2 to 7: results of iiPCR performed with 10¹, 10², 10³, 10⁴, 10⁵, 10⁶ copies of pTA-ISAV plasmid respectively. **FIG. 5D** shows the results of iiPCR test for sensitivity of primers ISAVF1 and ISAVR1, and probe ISAVP2 with serially diluted RNA of SAV; lane 1: result of iiPCR performed without RNA template (negative control); lanes 2 to 7: results of iiPCR performed with 10⁷, 10⁶, 10⁵, 10⁴, 10³, 10² -fold diluted total RNA of ISAV respectively. **FIG. 5E** shows the results of iiPCR test for specificity of primers ISAVF1 and ISAVR1, and probe ISAVP2; lane 1: result of iiPCR performed without RNA template (negative control); lanes 2 to 7: results of iiPCR performed with DNA/RNA template of SAV, IPVN, NNV, PRV, salmon muscle tissue, and pTA-ISAV plasmid respectively. **FIG. 5F** shows the results of real-time PCR test of ISAV, pTA-ISAV plasmid, with primers ISAVF1 and ISAVR1. "M" represents molecular weight marker; arrows indicate the target PCR products; "+" indicates fluorescent signal was detected in the sample, and "-" indicates fluorescent signal was not detected in the sample.

### DETAILED DESCRIPTION OF THE PREFERRED EMBODIMENT

In one aspect, the disclosure relates to a method for detecting a pathogen in coldwater fish. The method is insulated isothermal polymerase chain reaction (iiPCR). The disclosure relates to a method for detecting a pathogen in coldwater fish, comprising:
providing a sample potentially containing one or more nucleotide sequences of a pathogen in a coldwater fish;
providing an oligonucleotide primer pair defining the 5' ends of two complementary strands of a double stranded target sequence on the one or more nucleotide sequences of the pathogen;
providing an oligonucleotide probe having a sequence complementary to a segment of the double stranded target sequence, a fluorescer molecule attached to a first location on the oligonucleotide probe, and a quencher molecule attached to a second location on the oligonucleotide probe such that the quencher molecule substantially quenches the fluorescer molecule whenever the oligonucleotide probe is not hybridized to the segment of the double stranded target sequence and such that the fluorescer molecule is substantially unquenched whenever the oligonucleotide probe is hybridized to the segment of the double stranded target sequence;
providing a polymerase;
blending the sample, the oligonucleotide primer pair, the oligonucleotide probe, the polymerase, deoxyadenosine triphosphates (dATPs), deoxycytidine triphosphates (dCTPs), deoxyguanosine triphosphates (dGTPs), and deoxythymidine triphosphates (dTTPs) in a container to form a polymerase chain reaction (PCR) mixture;
subjecting the PCR mixture to insulated isothermal polymerase chain reaction (iiPCR) by heating the bottom of the container at a fixed temperature to form a PCR product; and
detecting the PCR product to identify the double stranded target sequence.
The disclosure also relates to a set of oligonucleotides and an oligonucleotide probe for detecting pathogens in coldwater fish. Further disclosed are embodiments, wherein a pair of oligonucleotides and/or an oligonucleotide probe disclosed herein are used in variations on the basic PCR technique, such as but not limited to, reverse-transcription PCR (RT-PCR), insulated isothermal PCR (iiPCR), real-time quantitative PCR (real-time PCR), nested PCR, and thermal asymmetric interlaced PCR (TAIL-PCR).

As used herein, the term "coldwater" refers to water areas on the northern and southern hemisphere with water temperatures ranging from freezing during winter and up to 15-20 °C during summer. Coldwater fish includes salmonids such as, but not limited to, Atlantic salmon, Coho salmon, Chinook salmon and Rainbow trout, furthermore marine species like halibut, cod and flounders. In some embodiments, the coldwater fish is salmon.

As used herein, the term "a pathogen in coldwater fish" refers to viral, bacterial and parasitic pathogen found in coldwater fish. Examples of pathogens in coldwater fish are such as but not limited to a) virus: IPNV (infectious pancreatic disease virus), SPDV (salmon pancreatic disease virus), ISAV (infectious salmon anemia virus), HSMIV (heart and skeletal muscle inflammation virus), IHNV (infectious hematopoietic necrosis virus), VHSV (viral hemorrhagic septicemia virus), VNNV (viral nervous necrosis virus); b) bacteria: BKD (bacterial kidney diseas) *Vibrio anguillarum, Vibrio salmonicida, Aeromonas salmonicida, Myxosoma cerebralis, Flavobacterium columnare Yersinia ruckeri, Candidatus Branchiomonas cysticola;* c) parasites: AGD (amoebic gill disease) and sea lice .

As used herein, the term "insulated isothermal polymerase chain reaction" (iiPCR) refers to a polymerase chain reaction in which spontaneous fluid convection in a tube is driven by temperature gradients that are formed simply by heating the vessel from the bottom at a fixed temperature. Consequently, reaction constituents are circulated through zones of temperature gradients, and the denaturation, annealing, and extension steps of PCR take place at the bottom, top, and middle zones of the tube, respectively. For a detailed description of the insulated isothermal PCR, see e.g., Chang et al., Biotech J. (2012) 7: 662-666; Tsai et al., J. Virol. Methods (2012) 181: 134-137; U.S. Patent Nos. 8,187,813 and 8,574,516; U.S. Publication Patent Nos: 2012/0094373, 2012/0309083, 2013/0023010, and 2013/0217112.

As used herein, the term "fluorescer molecule" refers to a substance or a portion thereof which is capable of exhibiting fluorescence in the detectable range. As used herein, the term "quencher molecule" refers to a substance or a portion thereof which is capable of quenching the fluorescence emitted by the fluorescer molecule when excited by a light source. In some embodiments, the terms "fluorescer molecule" and "quencher molecule" are the fluorescer molecule and the quencher molecule of the TaqMan™ assay (Applied Biosystems Inc., CA, US). For a detailed description of the TaqMan™ assay, reagents, and conditions for use therein, see, e.g., Holland et al., Proc. Natl. Acad. Sci, U.S.A. (1991) 88:7276- 7280; U.S. Patent Nos. 5,538,848, 5,723,591, 5,876,930, and 7,413,708.

Example of the fluorescer molecule includes, but not limited to, 3-(ε-carboxypentyl)-3'-ethyl-5,5'-dimethyloxa-carbocyanine (CYA); 6-carboxyfluorescein (FAM); 5,6-carboxyrhodamine-11O (R110); 6- carboxyrhodamine-6G (R6G); N',N',N',N'-tetramethyl-6-carboxyrhodamine (TAMRA); 6-carboxy-X-rhodamine (ROX); 2', 4', 5', T, - tetrachloro-4-7- dichlorofluorescein (TET); 2', 7- dimethoxy - 4', 5'- 6 carboxyrhodamine (JOE); 6- carboxy-2',4,4',5',7,7'-hexachlorofluorescein (HEX); ALEXA; Cy3 and Cy5. Example of the quencher molecule includes, but not limited to, 4-(4'-dimethylamino-phenylazo)-benzoic acid (Dabcyl), Black Hole Quencher 1 (BHQ1), Black Hole Quencher 2 (BHQ2), Black Hole Quencher 3 (BHQ3), tetramethylrhodamine (TAMRA). In some embodiments, the fluorescer molecule is 6-carboxyfluorescein (6-FAM), and the quencher molecule is dihydrocyclopyrroloindole tripeptide minor groove binder (MGB).

Unless otherwise defined herein, scientific and technical terms used in connection with the present disclosure shall have the meanings that are commonly understood by those of ordinary skill in the art. Generally, nomenclatures used in connection with, and techniques of biochemistry, enzymology, molecular and cellular biology, microbiology, genetics and protein and nucleic acid chemistry and hybridization described herein are those well-known and commonly used in the art. The methods and techniques of the present disclosure are generally performed according to conventional methods well known in the art and as described in various general and more specific references that are cited and discussed throughout the present specification unless otherwise indicated.

The present invention is further illustrated by the following Examples, which in no way should be construed as further limiting.

### EXAMPLES

### Example 1 Detection of Candidates Branchiomonas cysticola

The 16S ribosomal RNA gene of *Candidatus Branchiomonas cysticola* (GenBank accession No. JQ723599) was inserted in pUC57 cloning vector (Thermo, MA, US) to obtain pUC57-BC plasmid.

### 1. Conventional PCR with Primers BCF1 and BCR1

The 50 µl PCR mixture for conventional PCR contains diluted pUC57-BC plasmid (10¹⁰, 10⁹, 10¹, 10⁷, 10⁶, 10¹, 10⁴, 10¹, 10², 10¹, 10⁰ copies respectively), 0.01-2 µM forward primer BCF1 (SEQ ID NO: 1), 0.01-2 µM reverse primer BCR1 (SEQ ID NO: 2), 0.2 µM dNTP, and 1.25U Prime Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C and 30 seconds of extension at 72°C. The amplicons were analyzed subsequently on a 15% polyacrylamide gel in TAE buffer (40 mM Tris, 20 mM acetic acid, 1 mM EDTA) and visualized by ethidium bromide staining.

The results of the conventional PCR are presented in FIG. 1A. As shown in FIG. 1A, the bands in lanes 1 to 9 show that the primers BCF1 and BCR1 of the invention have correctly amplified the 90-bp target sequence, whereas no target sequence has been amplified in the negative control (lane 12). The results demonstrate that the primers can be used in conventional PCR amplification to detect the existence of *Candidatus Branchiomonas cysticola.*

### 2. iiPCR with Primers BCF1, BCR1 and Probe BCP1

The 50 µl PCR mixture, containing pUC57-BC plasmid (10⁰, 10¹, 10², 10³, 10⁴, 10⁵, 10⁶ copies respectively), 0.01-2 µM forward primer BCF1 (SEQ ID NO: 1), 0.01-2 µM reverse primer BCR1 (SEQ ID NO: 2), 0.01-2 µM probe BCP1 (5' FAM-CAGGCTTTCCTCTCCCA-MGB 3', SEQ ID NO: 3), 1X qPCR buffer, and 1-5 units of *Taq* DNA polymerase, was added in a reaction tube and incubated in an iiPCR machinefor a designated period of time (about 1 hour). The fluorescence of FAM in each sample was detected by the iiPCR machine. In addition, amplicons were analyzed subsequently on a 15% polyacrylamide gel in TAE buffer and visualized by ethidium bromide staining.

The results of the iiPCR are presented in FIG. 1B. As shown in FIG. 1B, the bands in lanes 2 to 7 show that the primers and probe of the invention have correctly amplified the 90-bp target sequence (indicated by arrows), whereas no target sequence has been amplified in the negative control (lane 1). In addition, significant probe hydrolysis was detected in the same mixtures by the iiPCR machine, whereas no fluorescent signal was detected in the negative control.

Furthermore, sensitivity evaluation of primer BCF1 (SEQ ID NO: 1), primer BCR1 (SEQ ID NO: 2) and probe BCP1 (SEQ ID NO: 3) was conducted by repeating the iiPCR analysis mentioned above six (6) times (n = 6). The result of the sensitivity test is shown in Table 1.

**Table 1 Result of Sensitivity Test of Primers BCF1, BCR1 and Probe BCP1 (n = 6)**

| DNA copies | Negative Control | 10¹ | 10² | 10³ | 10⁴ | 10⁵ | 10⁶ |
|---|---|---|---|---|---|---|---|
| Positive % | 0 | 17 | 50 | 100 | 100 | 100 | 100 |

Specificity evaluation of primer BCF1 (SEQ ID NO: 1), primer BCR1 (SEQ ID NO: 2) and probe BCP1 (SEQ ID NO: 3) was conducted by the following iiPCR analysis. The 50 µl PCR mixture, containing DNA extraction from salmon gill or DNA/RNA templates of different fish pathogens (SAV, IPNV, ISA, NNV, PRV, and pUC57-BC plasmid, respectively), 0.01-2 µM forward primer BCF1 (SEQ ID NO: 1), 0.01-2 µM reverse primer BCR1 (SEQ ID NO: 2), 0.01-2 µM probe BCP1 (5' FAM-CAGGCTTTCCTCTCCCA-MGB 3', SEQ ID NO: 3), 1X qPCR buffer, and 10 units of *Taq* DNA polymerase, was added in a reaction tube and incubated in an iiPCR machine for a designated period of time (about 1 hour). The fluorescence of FAM in each sample was detected by the iiPCR machine . In addition, amplicons were analyzed subsequently on a 15% polyacrylamide gel in TAE buffer and visualized by ethidium bromide staining.

The results of the iiPCR are presented in FIG. 1C. As shown in FIG. 1C, the 90-bp target sequence (indicated by arrows) has been correctly amplified only in the sample containing *Candidatus Branchiomonas cysticola* DNA template (lane 8), whereas no target sequence has been amplified in samples containing DNA/RNA template of fish gill, SAV, IPNV, ISA, NNV, or PRV (lanes 1 to 7). In addition, significant probe hydrolysis was detected in the same mixtures by the iiPCR machine, whereas no fluorescent signal was detected in lanes 1 to 7.

### 3. iiPCR with Primers BCF2, BCR2, BCF3, BCR3, BCF4, BCR4 and Probe BCP2

The 50 µl PCR mixture, containing pUC57-BC plasmid (10⁸ copies), 0.01-2 µM forward primer BCF2 (SEQ ID NO: 23) or BCF3 (SEQ ID NO: 25) or BCF4 (SEQ ID NO: 27), 0.01-2 µM reverse primer BCR2 (SEQ ID NO: 24) or BCR3 (SEQ ID NO: 26) or BCR4 (SEQ ID NO: 28), 0.01-2 µM probe BCP2 (5' FAM-CGGCGTGCCTGAGAA-MGB 3', SEQ ID NO: 29), 1X qPCR buffer, and 5 units of *Taq* DNA polymerase, was added in a reaction tube and incubated in an iiPCR machine for a designated period of time (about 1 hour). The fluorescence of FAM in each sample was detected by the iiPCR machine. In addition, amplicons were analyzed subsequently on a 15% polyacrylamide gel in TAE buffer and visualized by ethidium bromide staining.

The results of the iiPCR are presented in FIG. 1D. As shown in FIG. 1D, the bands in lanes 2, 4, and 6 show that the primers BCF2 (SEQ ID NO: 23) and BCR2 (SEQ ID NO: 24), BCF3 (SEQ ID NO: 25) and BCR3 (SEQ ID NO: 26), BCF4 (SEQ ID NO: 27) and BCR4 (SEQ ID NO: 28) have correctly amplified the 100-bp, 75-bp, and 100-bp target sequences (indicated by arrows) respectively, whereas no target sequence has been amplified in the negative controls (lanes 1, 3, and 5). In addition, significant probe hydrolysis was detected in the same mixtures by the iiPCR machine, whereas no fluorescent signal was detected in the negative controls. The results also demonstrate that the primers BCF2, BCR2, BCF3, BCR3, BCF4, BCR4 and the probe BCP2 can be used in iiPCR amplification to detect the existence of *Candidatus*

### Branchiomonas cysticola.

Furthermore, sensitivity evaluation of primer BCF3 (SEQ ID NO: 25), primer BCR3 (SEQ ID NO: 26) and probe BCP2 (SEQ ID NO: 29) was conducted by repeating the iiPCR analysis mentioned above seven (7) times (n = 7) with different copies (10⁰, 10¹, 10², 10³, 10⁴, 10⁵, 10⁶ copies respectively) of DNA template pUC57-BC plasmid. The result of the sensitivity test is shown in Table 2 and FIG. 1E.

**Table 2 Result of Sensitivity Test of Primers BCF3, BCR3 and Probe BCP2 (n = 7)**

| DNA copies | 10¹ | 10² | 10³ | 10⁴ | 10⁵ | 10⁶ |
|---|---|---|---|---|---|---|
| Positive % | 42.9 | 100 | 100 | 100 | 100 | 100 |

As shown in FIG. 1E, the bands in lanes 3 to 8 show that the primers and probe of the invention have correctly amplified the 75-bp target sequence (indicated by arrows), whereas no target sequence has been amplified in the negative control and the sample with 10⁰ copy of pUC57-BC plasmid (lanes 1 and 2). In addition, significant probe hydrolysis was detected in the same mixtures by the iiPCR machine, whereas no fluorescent signal was detected in lanes 1 and 2.

### 4. Real-time PCR with Primers BCF3, BCR3 and Probe BCP2

Real-time PCR assay were carried out in a real time PCR machine (such as, but not limited to ABI StepOnePlus™; Applied BioSystem, Life Technologies, CA, USA) using diluted pUC57-BC plasmid (10¹, 10², 10¹, 10⁴, 10⁵, 10⁶ copies). Real-time PCR assays were performed with 2 µl pUC57-BC plasmid, 0.01-2 µM forward primer BCF3 (SEQ ID NO: 25), 0.01-2 µM reverse primer BCR3 (SEQ ID NO: 26), and 0.01-2 µM probe BCP2 (5' FAM-CGGCGTGCCTGAGAA-MGB 3', SEQ ID NO: 29) in a total volume of 20 µl by using a commercial RT-PCR Kit (such as, but not limited to, OneStep PrimeScript™ RT-PCR Kit; Takara Bio Inc., Japan). The program included an incubation period at 42°C 5min, 94°C for 10 sec, and 40 cycles of 94°C for 10 sec and 60°C for 30 min. Fluorescence measurements were recorded at the 60°C step.

As shown in FIG. 1F, the standard curve for the serial dilutions (10-fold) of the pUC57-BC plasmid was calculated for the real-time PCR assays. At least 10 copies of pUC57-BC plasmid can be detected. The R² value of the standard curve is 0.994, indicating the primers and the probe of the present invention can be used in real-time PCR to produce reliable outcomes.

The results demonstrate that the primers and the probes of the invention can be used in iiPCR amplification to detect the existence of *Candidatus Branchiomonas cysticola* with high sensitivity and specificity.

### Example 2 Detection of Piscine Reovirus (PRV)

The segment L1 of piscine reovirus (PRV, GenBank accession No. GU994013) was inserted in pUC57 cloning vector (Thermo) to obtain pUC57-PRV plasmid.

In addition, in vitro transcriptional RNA template of PRV was prepared by transcribing the DNA sequence of the PRV segment L1 with a commercial kit (such as, but not limited to, MAXIscript® Kit; Thermo Fisher Scientific, MA, USA).

### 1. Conventional PCR with Primers PRVF1 and PRVR1

The 50 µl PCR mixture for conventional PCR contains diluted pUC57-PRV plasmid (10⁶, 10⁵, 10⁴, 10³, 10², 10¹ copies respectively), 0.01-2 µM forward primer PRVF1 (SEQ ID NO: 4), 0.01-2 µM reverse primer PRVR1 (SEQ ID NO: 5), 0.2 µM dNTP, and 1.25U Prime Taq DNA polymerase. The amplification was performed in a thermal cycler (such as, but not limited to, Astec Co. Ltd.) and consisted of one initial cycle of denaturation for 3 minutes at 94°C and 35 cycles of 30 seconds at 94°C, 30 seconds at 60°C and 30 seconds of extension at 72°C. The amplicons were analyzed subsequently on a 15% polyacrylamide gel in TAE buffer and visualized by ethidium bromide staining.

The results of the conventional PCR are presented in FIG. 2A. As shown in FIG. 2A, the bands in lanes 1 to 3 show that the primers PRVF1 and PRVR1 of the invention have correctly amplified the 86-bp target sequence, whereas no target sequence has been amplified in the negative control (lane 7). The results demonstrate that the primers can be used in conventional PCR amplification to detect the existence of piscine reovirus (PRV).

### 2. iiPCR with Primers PRV1, PRVR1, PRVF2, PRVR2, PRVF3, PRVF4, PRVR4 and Probe PRVP1

The 50 µl PCR mixture, containing pUC57-PRV plasmid, 0.01-2 µM forward primer PRVF1 (SEQ ID NO: 4) or PRVF2 (SEQ ID NO: 30) or PRVF3 (SEQ ID NO: 32) or PRVF4 (SEQ ID NO: 33), 0.01-2 µM reverse primer PRVR1 (SEQ ID NO: 5) or PRVR2 (SEQ ID NO: 31) or PRVR4 (SEQ ID NO: 34), 0.01-2 µM probe PRVP1 (5' FAM-CTCCAGGAGTCATTGTC-MGB 3', SEQ ID NO: 6), 1X qPCR buffer, and 5 units of *Taq* DNA polymerase, was added in a reaction tube and incubated in an iiPCR machine for a designated period of time (about 1 hour). The fluorescence of FAM in each sample was detected by the iiPCR machine. In addition, amplicons were analyzed subsequently on a 15% polyacrylamide gel in TAE buffer and visualized by ethidium bromide staining.

The results of the iiPCR are presented in FIG. 2B and FIG. 2C. As shown in FIG. 2B, the bands in lanes 1 and 3 show that the primers PRVF1 (SEQ ID NO: 4) and PRVR1 (SEQ ID NO: 5) have correctly amplified the 86-bp target sequence (indicated by arrows), whereas no target sequence has been amplified in the negative controls (lanes 2 and 4). In addition, significant probe hydrolysis was detected in the same mixtures by the iiPCR machine, whereas no fluorescent signal was detected in the negative controls.

As shown in FIG. 2C, the bands in lanes 2, 4, 6, 8, 10, and 12 show that the primers PRVF2 (SEQ ID NO: 30) and PRVR2 (SEQ ID NO: 31), PRVF3 (SEQ ID NO: 32) and PRVR2 (SEQ ID NO: 31), PRVF4 (SEQ ID NO: 33) and PRVR4 (SEQ ID NO: 34) have correctly amplified the 89-bp, 83-bp, and 100-bp target sequences (indicated by arrows) respectively, whereas no target sequence has been amplified in the negative controls (lanes 1, 3, 5, 7, 9, and 11). In addition, significant probe hydrolysis was detected in the same mixtures by the iiPCR machine, whereas no fluorescent signal was detected in the negative controls.

Furthermore, sensitivity evaluation of primers PRVF2 (SEQ ID NO: 30) and PRVR2 (SEQ ID NO: 31), and PRVF3 (SEQ ID NO: 32) and PRVR2 (SEQ ID NO: 31), and probe PRVP1 (SEQ ID NO: 6) was conducted by repeating the iiPCR analysis mentioned above with different copies (10¹, 10², 10³, 10⁴, 10⁵, 10⁶ copies respectively) of in vitro transcriptional RNA template of PRV The result of the sensitivity test is shown in FIG. 2D (for primers PRVF2, PRVR2 and probe PRV1) and FIG. 2E and Table 3 (for primers PRVF3, PRVR2 and probe PRV1), respectively.

As shown in FIG. 2D, the bands in lanes 4 to 7 show that the primers PRVF2 and PRVR2 and probe PRVP1 have correctly amplified the 89-bp target sequence (indicated by arrows), whereas no target sequence has been amplified in the negative control and the sample with 10¹ and 10² copies of in vitro transcriptional RNA (lanes 1, 2, and 3). In addition, significant probe hydrolysis was detected in the same mixtures by the iiPCR machine, whereas no fluorescent signal was detected in lanes 1, 2, and 3.

As shown in FIG. 2E, the bands in lanes 3 to 7 show that the primers PRVF3, PRVR2 and probe PRVP1 have correctly amplified the 83-bp target sequence (indicated by arrows), whereas no target sequence has been amplified in the negative control and the sample with 10¹ copy of in vitro transcriptional RNA (lanes 1 and 2). In addition, significant probe hydrolysis was detected in the same mixtures by the iiPCR machine, whereas no fluorescent signal was detected in lanes 1 and 2. The statistical data of eight (8) repeats of the sensitivity test is shown in Table 3.

**Table 3 Result of Sensitivity Test of Primers PRVF3, PRVR2 and Probe PRVP1 (n = 8)**

| RNA copies | 10¹ | 10² | 10³ | 10⁴ | 10⁵ | 10⁶ |
|---|---|---|---|---|---|---|
| Positive % | 1.25 | 3.75 | 75 | 100 | 100 | 100 |

### 3. iiPCR with Primers PRVF5, PRVR5, and Probe PRVP2

The 50 µl PCR mixture, containing pUC57-PRV plasmid (10¹, 10², 10¹, 10⁴, 10¹, 10⁶ copies respectively) or in vitro transcriptional RNA template of PRV (10¹, 10², 10³, 10⁴, 10⁵, 10⁶ copies respectively), 0.01-2 µM forward primer PRVF5 (SEQ ID NO: 35), 0.01-2 µM reverse primer PRVR5 (SEQ ID NO: 36), 0.01-2 µM probe PRVP2 (5' FAM-CAATGGGATGCTAACACTC-MGB 3', SEQ ID NO: 37), 1X qPCR buffer, and 5 units of *Taq* DNA polymerase, was added in a reaction tube and incubated in an iiPCR machine for a designated period of time (about 1 hour). The fluorescence of FAM in each sample was detected by the iiPCR machine. In addition, amplicons were analyzed subsequently on a 15% polyacrylamide gel in TAE buffer and visualized by ethidium bromide staining. The results of the iiPCR are presented in FIG. 2F (for DNA plasmid), FIG. 2G, and Table 4 (for in vitro transcriptional RNA).

As shown both in FIG. 2F and FIG. 2G, the bands in lanes 3 to 7 show that the primers of the invention have correctly amplified the 72-bp target sequence (indicated by arrows), whereas no target sequence has been amplified in the negative control and the sample with 10¹ copy of pUC57-PRV plasmid (lanes 1 and 2 in FIG. 2F) or in vitro transcriptional RNA template of PRV (lanes 1 and 2 in FIG. 2G). In addition, significant probe hydrolysis was detected in the same mixtures by the iiPCR machine , whereas no fluorescent signal was detected in lanes 1 and 2 in FIG. 2F or FIG. 2G. The statistical data of four (4) repeats of the sensitivity test with in vitro transcriptional RNA template of PRV is shown in Table 4.

**Table 4 Result of Sensitivity Test of Primers PRVF5, PRVR5 and Probe PRVP2 (n = 4)**

| RNA copies | 10¹ | 10² | 10³ | 10⁴ | 10⁵ | 10⁶ |
|---|---|---|---|---|---|---|
| Positive % | 0 | 75 | 100 | 100 | 100 | 100 |

The results demonstrate that the primers and the probes of the invention can be used in iiPCR amplification to detect the existence of piscine reovirus (PRV) with high sensitivity.

### Example 3 Detection of Infectious Pancreatic Necrosis Virus (IPNV)

The segment A of infectious pancreatic necrosis virus (IPNV, GenBank accession No. AY379740) was inserted in T&A™ cloning vector (Yeastern biotech, Taiwan) to obtain pTA-IPNV plasmid.

In vitro transcriptional RNA template of IPNV was prepared by transcribing the DNA sequence of the IPNV segment A with a commercial kit (such as, but not limited to, MAXIscript® Kit; Thermo Fisher Scientific, MA, USA).

In addition, virus sample from salmon from a fish farm were collected and tested to evaluate the performance of the iiPCR and real-time PCR assays from field samples. The fish in the salmon farms were diagnosed to suffer from infectious pancreatic necrosis virus (IPNV). RNA extraction from tissues was performed by using a commercial kit (such as, but not limited to, FavorPrep™ Viral Nucleic Acid Extraction kit; Favorgen Biotech Corp., Taiwan). The purity of the total RNA was evaluated by measuring the absorbance ration at 260/280 nm.

### 1. Conventional PCR

The 50 µl PCR mixture for conventional PCR contains diluted pTA-IPNV plasmid (10⁵, 10⁴, 10³, 10², 10¹ copies respectively), 0.01-2 µM forward primer IPNVF1 (SEQ ID NO: 7), 0.01-2 µM reverse primer IPNVR1 (SEQ ID NO: 8), 0.2 µM dNTP, and 1.25U Prime Taq DNA polymerase. The amplification was performed in a thermal cycler (such as, but not limited to, Astec Co. Ltd.) and consisted of one initial cycle of denaturation for 3 minutes at 94°C and 35 cycles of 30 seconds at 94°C, 30 seconds at 60°C and 30 seconds of extension at 72°C. The amplicons were analyzed subsequently on a 15% polyacrylamide gel in TAE buffer and visualized by ethidium bromide staining.

The results of the conventional PCR are presented in FIG. 3A. As shown in FIG. 3A, the bands in lanes 1 to 4 show that the primers and probe of the invention have correctly amplified the 83-bp target sequence, whereas no target sequence has been amplified in the negative control (lane 6). The results demonstrate that the primers can be used in conventional PCR amplification to detect the existence of infectious pancreatic necrosis virus (IPNV).

### 2. RT-PCR

RT-PCR was performed by using a commercial kit (such as, but not limited to, One step RT-PCR kit; LTK Biolab, Taiwan) with the total RNA extraction from fish suffering from infectious pancreatic necrosis virus (IPNV), 0.01-2 µM forward primer IPNVF1 (SEQ ID NO: 7), and 0.01-2 µM reverse primer IPNVR1 (SEQ ID NO: 8). The reverse transcription reaction was carried out at 42°C for 30min, followed by a PCR program of 10 minutes at 94°C and 35 cycles of 30 seconds at 94°C, 30 seconds at 60°C and 30 seconds of extension at 72°C. The amplicons were analyzed subsequently on a 15% polyacrylamide gel in TAE buffer and visualized by ethidium bromide staining.

The results of the RT-PCR are presented in FIG. 3B. As shown in FIG. 3B, the bands in lanes 1 to 3 show that the primers of the invention have correctly amplified the 83-bp target sequence, whereas no target sequence has been amplified in the negative control (lane 7). The results demonstrate that the primers can also be used in RT-PCR amplification to detect the existence of infectious pancreatic necrosis virus (IPNV).

### 3. iiPCR

The 50 µl PCR mixture, containing pTA-IPNV plasmid, 0.01-2 µM forward primer IPNVF1 (SEQ ID NO: 7), 0.01-2 µM reverse primer IPNVR1 (SEQ ID NO: 8) or IPNVR2 (SEQ ID NO: 9), 0.01-2 µM probe IPNVP1 (5' FAM-ACGCTCAACGCTGCCA-MGB 3', SEQ ID NO: 10), 1X qPCR buffer, and 5 units of *Taq* DNApolymerase, was added in a reaction tube and incubated in an iiPCR machine for a designated period of time (about 1 hour). The fluorescence of FAM in each sample was detected by the iiPCR machine. In addition, amplicons were analyzed subsequently on a 15% polyacrylamide gel in TAE buffer and visualized by ethidium bromide staining.

The results of the iiPCR are presented in FIG. 3C. As shown in FIG. 3C, the bands in lanes 1 and 3 show that the primers and probe of the invention have correctly amplified the 83-bp target sequence (primers IPNVF1 and IPNVR1) and 88-bp target sequence (primers IPNVF1 and IPNVR2) respectively, whereas no target sequence has been amplified in the negative controls (lanes 1 and 3). In addition, significant probe hydrolysis was detected in the same mixtures by the iiPCR machine, whereas no fluorescent signal was detected in the negative controls.

Furthermore, sensitivity evaluation of primer IPNV (SEQ ID NO: 7), primer IPNVR1 (SEQ ID NO: 8) and probe IPNV (SEQ ID NO: 10) was conducted by iiPCR analysis with pTA-IPNV DNA plasmid (10¹, 10², 10¹, 10⁴, 10⁵ copies respectively), in vitro transcriptional RNA template of IPNV (10¹, 10², 10³, 10⁴, 10⁵, 10⁶ copies respectively), or total RNA extraction from fish suffering from IPNV (original stock and 10⁻³, 10⁻⁴, 10⁻⁵, 10⁻⁶, 10⁻⁷ dilution, respectively). For iiPCR analysis with total RNA extraction, 50 U MMLV RTase and 4 U RNase inhibitor were also added in the reactions. The results of the sensitivity tests are shown in FIGs. 3D, 3E, 3F.

As shown in FIG. 3D, the bands in lanes 1 to 5 show that the primers of the invention have correctly amplified the 83-bp target sequence (indicated by arrows), whereas no target sequence has been amplified in the negative control (lane 6). In addition, significant probe hydrolysis was detected in the same mixtures by the iiPCR machine, whereas no fluorescent signal was detected in lane 6.

As shown in FIG. 3E, the bands in lanes 4 to 7 show that the primers of the invention have correctly amplified the 83-bp target sequence (indicated by arrows), whereas no target sequence has been amplified in the negative control and the sample with 10¹ and 10² copies of in vitro transcriptional RNA template of IPNV (lanes 1, 2 and 3). In addition, significant probe hydrolysis was detected in the same mixtures by the iiPCR machine, whereas no fluorescent signal was detected in lanes 1, 2, and 3.

As shown in FIG. 3F, the bands in lanes 1, 2, 3, and 8 show that the primers of the invention have correctly amplified the 83-bp target sequence, whereas no target sequence has been amplified in the 10⁻⁵, 10⁻⁶, 10⁻⁷ diluted RNA samples and the negative control (lanes 4, 5, 6, and 7). In addition, significant probe hydrolysis was detected in the same mixtures by the iiPCR machine, whereas no fluorescent signal was detected in the negative controls and the 10⁻⁵, 10⁻⁶, 10⁻⁷ diluted RNA samples. The statistical data of five (5) repeats of the sensitivity test with RNA samples of PRV is shown in Table 5.

**Table 5 Result of Sensitivity Test of Primers IPNV1, IPNVR1 and Probe IPNV (n = 5)**

| RNA dilution | 10⁻⁷ | 10⁻⁶ | 10⁻⁵ | 10⁻⁴ | 10⁻³ | 10⁻² | 10⁻¹ |
|---|---|---|---|---|---|---|---|
| Positive % | 0 | 0 | 0 | 20 | 100 | 100 | 100 |

Specificity evaluation of primer IPNVF1 (SEQ ID NO: 7), primer IPNVR1 (SEQ ID NO: 8) and probe IPNVP1 (SEQ ID NO: 10) was conducted by the iiPCR analysis mentioned above with DNA extraction from salmon muscle tissue and DNA/RNA templates of different fish pathogens (ISAV, SAV, NNV, PRV, and IPNV, respectively).

The results of the iiPCR are presented in FIG. 3G. As shown in FIG. 3G, the 83-bp target sequence (indicated by arrows) has been correctly amplified only in the sample containing IPNV RNA template (lane 7), whereas no target sequence has been amplified in samples containing DNA/RNA templates of ISAV, SAV, NNV, PRV, DNA extraction of Salmon muscle tissue, and negative control (lanes 1 to 6). In addition, significant probe hydrolysis was detected in the same mixtures by the iiPCR machine, whereas no fluorescent signal was detected in lanes 1 to 6.

### 4. Real-time PCR

Real-time PCR assay were carried out in a real time PCR machine (such as, but not limited to, an ABI StepOnePlus™ real time PCR machine; Applied BioSystem, Life Technologies, CA, USA) using diluted pTA-IPNV plasmid (10¹, 10², 10¹, 10⁴, 10⁵, 10⁶ copies). Real-time PCR assays were performed with 2 µl pTA-IPNV plasmid, 0.01-2µM forward primer IPNVF1 (SEQ ID NO: 7), 0.01-2µM reverse primer IPNVR1 (SEQ ID NO: 8), and 0.01-2µM probe IPNVP1 (5' FAM-ACGCTCAACGCTGCCA-MGB 3', SEQ ID NO: 10) in a total volume of 20 ul by using a commercial kit (such as, but not limited to, OneStep PrimeScript™ RT-PCR Kit; Takara Bio Inc., Japan). The program included an incubation period at 42°C 5min, 94°C for 10 sec, and 40 cycles of 94°C for 10 sec and 60°C for 30 min. Fluorescence measurements were recorded at the 60°C step.

As shown in FIG. 3H, the standard curve for the serial dilutions (10-fold) of the pTA-IPNV plasmid was calculated for the real-time PCR assays. At least 10 copies of pTA-IPNV plasmid can be detected. The R² value of the standard curve is 0.99795, indicating the primers and the probe of the present invention can be used in real-time PCR to produce reliable outcomes.

The results demonstrate that the primers and the probe can be used in iiPCR amplification to detect the existence of infectious pancreatic necrosis virus (IPNV) with high sensitivity and specificity.

### Example 4 Detection of Salmonid Alphavirus (SAV)

A 984-bp partial sequence of salmonid alphavirus (SAV, GenBank accession No. KC122926, from nt 33 to 1016) was inserted in T&A™ cloning vector (Yeastern biotech) to obtain pTA-SAV plasmid.

In vitro transcriptional RNA template of SAV was prepared by transcribing the DNA sequence of the 984-bp partial sequence of SAV with a commercial kit (such as, but not limited to, MAXIscript® Kit; Thermo Fisher Scientific, MA, USA).

In addition, heart tissues from salmon from a fish farm were collected and tested to evaluate the performance of the iiPCR assays from field samples. The fish in the salmon farms were diagnosed to suffer from salmonid alphavirus (SAV). RNA extraction from tissues was performed by using a commercial kit (such as, but not limited to, FavorPrep™ Viral Nucleic Acid Extraction kit; Favorgen Biotech Corp., Taiwan). The purity of the total RNA was evaluated by measuring the absorbance ration at 260/280 nm, and RNA quality was checked on an agarose (1%) gel in TAE buffer and visualized by ethidium bromide staining.

### 1. iiPCR

The 50 µl PCR mixture, containing pTA-SAV plasmid, 0.01-2 µM forward primer SAVF1, SAVF2, or SAVF3 (SEQ ID NOs: 11, 12, or 13 respectively), 0.01-2 µM reverse primer SAVR1, SAVR2, or SAVR3 (SEQ ID NOs: 14, 15, or 16 respectively), 0.01-2 µM probe SAVP1 (5' FAM-CGTGAGTTGTAAAGTAAAG-MGB 3', SEQ ID NO: 17), 1X qPCR buffer, and 5 units of *Taq* DNA polymerase, was added in a reaction tube and incubated in an iiPCR machine for a designated period of time (about 1 hour). The fluorescence of FAM in each sample was detected by the iiPCR machine. In addition, amplicons were analyzed subsequently on a 15% polyacrylamide gel in TAE buffer and visualized by ethidium bromide staining.

The results of the iiPCR are presented in FIG. 4A. As shown in FIG. 4A, the bands in lanes 2, 4, and 6 show that the primers and probe of the invention have correctly amplified the 70-bp target sequence (primers SAVF1 and SAVR1), 85-bp target sequence (primers SAVF2 and SAVR2), and 70-bp target sequence (primers SAVF3 and SAVR3) respectively (indicated by arrows), whereas no target sequence has been amplified in the negative controls (lanes 1, 3, and 5). In addition, significant probe hydrolysis was detected in the same mixtures by the iiPCR machine, whereas no fluorescent signal was detected in the negative controls.

Furthermore, sensitivity evaluation of primer SAVF1 (SEQ ID NO: 11), primer SAVR1 (SEQ ID NO: 14) and probe SAVP1 (SEQ ID NO: 17) was conducted by iiPCR analysis with pTA-SAV DNA plasmid (10¹, 10², 10³, 10⁴, 10⁵ copies respectively), in vitro transcriptional RNA template of SAV (10¹, 10², 10¹, 10⁴, 10⁵, 10⁶ copies respectively), or total RNA extraction from fish suffering from IPNV (original stock and 10⁻¹, 10⁻³, 10⁻⁴, 10⁻⁵, 10⁻⁶ dilution, respectively). For iiPCR analysis with total RNA extraction, 50 U MMLV RTase and 4 U RNase inhibitor were also added in the reactions. The results of the sensitivity tests are shown in FIGs. 4B, 4C, and 4D.

As shown in FIG. 4B, the bands in lanes 1 to 4 show that the primers of the invention have correctly amplified the 70-bp target sequence (indicated by arrows), whereas no target sequence has been amplified in the sample with 10¹ copies of pTA-SAV DNA plasmid and the negative control (lanes 5 and 6). In addition, significant probe hydrolysis was detected in the same mixtures by the iiPCR machine, whereas no fluorescent signal was detected in lanes 5 and 6.

As shown in FIG. 4C, the bands in lanes 3 to 7 show that the primers of the invention have correctly amplified the 70-bp target sequence (indicated by arrows), whereas no target sequence has been amplified in the negative control and the sample with 10¹ copies of in vitro transcriptional RNA template of SAV (lanes 1 and 2). In addition, significant probe hydrolysis was detected in the same mixtures by the iiPCR machine , whereas no fluorescent signal was detected in lanes 1 and 2.

As shown in FIG. 4D, the bands in lanes 1 to 6 show that the primers of the invention have correctly amplified the 70-bp target sequence from the extracted RNA, whereas no target sequence has been amplified in the negative controls (lane 7). In addition, significant probe hydrolysis was detected in the same mixtures by the iiPCR machine , whereas no fluorescent signal was detected in the negative controls. The statistical data of five (5) repeats of the sensitivity test with RNA samples of SAV is shown in Table 6.

**Table 6 Result of Sensitivity Test of Primers SAVF1, SAVR1 and Probe SAVP1 (n = 5)**

| RNA dilution | 10⁻⁷ | 10⁻⁶ | 10⁻⁵ | 10⁻⁴ | 10⁻³ | 10⁻² | 10⁻¹ |
|---|---|---|---|---|---|---|---|
| Positive % | 0 | 20 | 60 | 60 | 100 | 100 | 100 |

### 2. Real-time PCR

Real-time PCR assay was carried out in an ABI StepOnePlus™ real time PCR machine (Applied BioSystem) using diluted pTA-SAV plasmid (10¹, 10², 10¹, 10⁴, 10¹, 10⁶ copies). Real-time PCR assays were performed with 2 µl pTA-SAV plasmid, 0.01-2 µM forward primer SAVF1 (SEQ ID NO: 11), 0.01-2 µM reverse primer SAVR1 (SEQ ID NO: 14), and 0.01-2 µM probe SAVP1 (5' FAM-CGTGAGTTGTAAAGTAAAG-MGB 3', SEQ ID NO: 17) in a total volume of 20 ul by using a commercial kit (such as, but not limited to, OneStep PrimeScript™ RT-PCR Kit; Takara Bio Inc., Japan). The program included an incubation period at 42°C 5min, 94°C for 10 sec, and 40 cycles of 94°C for 10 sec and 60°C for 30 min. Fluorescence measurements were recorded at the 60°C step.

As shown in FIG. 4E, the standard curve for the serial dilutions (10-fold) of the pTA-SAV plasmid was calculated for the real-time PCR assays. At least 10 copies of pTA-SAV plasmid can be detected. The R² value of the standard curve is 0.99046, indicating the primers and the probe of the present invention can be used in real-time PCR to produce reliable outcomes.

The results demonstrate that the primers and the probe can be used in iiPCR amplification to detect the existence of salmonid alphavirus (SAV) with high sensitivity.

### Example 5 Detection of Infectious Salmon Anemia Virus (ISAV)

A non-structural protein and matrix protein sequence of infectious salmon anemia virus (ISAV, GenBank accession No. DQ785286) was inserted in T&A™ cloning vector (Yeastern biotech) to obtain pTA-ISAV plasmid.

In addition, heart tissues from salmon from a fish farm were collected and tested to evaluate the performance of the iiPCR assays from field samples. The fish in the salmon farms were diagnosed to suffer from infectious salmon anemia virus (ISAV). RNA extraction from tissues was performed by using a commercial kit (such as, but not limited to, FavorPrep™ Viral Nucleic Acid Extraction kit; Favorgen Biotech Corp., Taiwan). The purity of the total RNA was evaluated by measuring the absorbance ration at 260/280 nm, and RNA quality was checked on an agarose (1%) gel in TAE buffer and visualized by ethidium bromide staining.

### 1. Conventional PCR

The 50 µl PCR mixture for conventional PCR contains diluted pTA-ISAV plasmid (10⁵, 10⁴, 10³, 10², 10¹ copies respectively), 0.01-2 µM forward primer ISAVF1 (SEQ ID NO: 18), 0.01-2 µM reverse primer ISAVR1 (SEQ ID NO: 20), 0.2 µM dNTP, and 1.25U Prime Taq DNApolymerase. The amplification was performed in a thermal cycler (such as, but not limited to, Astec Co. Ltd.) and consisted of one initial cycle of denaturation for 3 minutes at 94°C and 35 cycles of 30 seconds at 94°C, 30 seconds at 60°C and 30 seconds of extension at 72°C. The amplicons were analyzed subsequently on a 15% polyacrylamide gel in TAE buffer and visualized by ethidium bromide staining.

The results of the conventional PCR are presented in FIG. 5A. As shown in FIG. 5A, the bands in lanes 1 to 3 show that the primers of the invention have correctly amplified the 92-bp target sequence, whereas no target sequence has been amplified in the negative control (lane 6). The results demonstrate that the primers and the probe can be used in conventional PCR amplification to detect the existence of infectious salmon anemia virus (ISAV).

### 2. iiPCR

The 50 µl PCR mixture, containing pTA-ISAV plasmid, 0.01-2 µM forward primer ISAVF1 or ISAVF2 (SEQ ID NOs: 18 or 19 respectively), 0.01-2 µM reverse primer ISAVR1 or ISAVR2 (SEQ ID NOs: 20 or 21 respectively), 0.01-2 µM probe ISAVP1 (5' FAM-CGACGATGACTCTCTACTGTGT-MGB 3', SEQ ID NO: 22) or ISAVP2 (5' FAM-CGATGACTCTCTACTGTGTGA-MGB 3', SEQ ID NO: 38), 1X qPCR buffer, and 5 units of *Taq* DNA polymerase, was added in a reaction tube and incubated in an iiPCR machine for a designated period of time (about 1 hour). The fluorescence of FAM in each sample was detected by the iiPCR machine. In addition, amplicons were analyzed subsequently on a 15% polyacrylamide gel in TAE buffer and visualized by ethidium bromide staining.The results of the iiPCR are presented in FIG. 5B and FIG. 5C.

As shown in FIG. 5B, the bands in lanes 1, 3, 5, and 7 show that the primers and probe ISAVP1 (SEQ ID NO: 22) of the invention have correctly amplified the 92-bp target sequence (primers ISAVF1 and ISAVR1), 93-bp target sequence (primers ISAVF1 and ISAVR2), 128-bp target sequence (primers ISAVF2 and ISAVR2), and 127-bp target sequence (primers ISAVF2 and ISAVR1) respectively, whereas no target sequence has been amplified in the negative controls (lanes 1, 3, 5, and 7). In addition, significant probe hydrolysis was detected in the same mixtures by the iiPCR machine, whereas no fluorescent signal was detected in the negative controls.

As shown in FIG. 5C, the bands in lanes 5, 6, and 7 show that the primers ISAVF1 (SEQ ID NO: 18) and ISAVR1 (SEQ ID NO: 20) and probe ISAVP2 (5' FAM-CGATGACTCTCTACTGTGTGA -MGB 3', SEQ ID NO: 38) of the invention have correctly amplified the 92-bp target sequence in samples with 10⁴, 10⁵, 10⁶ copies of pTA-ISAV plasmid, whereas no target sequence has been amplified in the negative controls and in samples with 10¹, 10², 10³ copies of pTA-ISAV plasmid (lanes 1 to 4). In addition, significant probe hydrolysis was detected in the same mixtures by the iiPCR machine, whereas no fluorescent signal was detected in lanes 1 to 4.

Sensitivity evaluation of primers ISAVF1 (SEQ ID NO: 18) and ISAVR1 (SEQ ID NO: 20) and probe ISAVP2 (5' FAM- CGATGACTCTCTACTGTGTGA-MGB 3', SEQ ID NO: 38) was also conducted by iiPCR analysis with total RNA extraction from fish suffering from ISAV (10⁻², 10⁻³, 10⁻⁴, 10⁻⁵, 10⁻⁶, 10⁻⁷ dilution, respectively). For iiPCR analysis with total RNA extraction, 50 U MMLV RTase and 4 U RNase inhibitor were also added in the reactions. The iiPCR analysis was repeated five (5) times (n = 5). The results of the iiPCR are presented in Table 7 and FIG. 5D.

**Table 7 Result of Sensitivity Test of Primers ISAVF1, ISAVR1 and Probe ISAVP1 (n = 5)**

| RNA dilution | 10⁻⁷ | 10⁻⁶ | 10⁻⁵ | 10⁻⁴ | 10⁻³ | 10⁻² |
|---|---|---|---|---|---|---|
| Positive % | 0 | 20 | 60 | 100 | 100 | 100 |

As shown in FIG. 5D, the bands in lanes 4 to 7 show that the primers and probe of the invention have correctly amplified the 92-bp target sequence, whereas no target sequence has been amplified in the negative control and the 10⁻⁶, 10⁻⁷ diluted RNA samples (lanes 1 to 3). In addition, significant probe hydrolysis was detected in the same mixtures by the iiPCR machine, whereas no fluorescent signal was detected in the negative controls and the 10⁻⁶, 10⁻⁷ diluted RNA samples.

Specificity evaluation of primer ISAVF1 (SEQ ID NO: 18) and ISAVR1 (SEQ ID NO: 20) and probe ISAVP2 (SEQ ID NO: 38) was conducted by the iiPCR analysis mentioned above with DNA/RNA templates of different fish pathogens (SAV, IPNV, NNV, PRV, Salom, and ISAV, respectively).

The results of the iiPCR are presented in FIG. 5E. As shown in FIG. 5E, the 92-bp target sequence (indicated by arrows) has been correctly amplified only in the sample containing ISAV RNA template (lane 7), whereas no target sequence has been amplified in negative control, samples containing DNA/RNA templates of SAV, IPNV, NNV, PRV, and DNA extraction from salmon muscle tissue (lanes 1 to 6). In addition, significant probe hydrolysis was detected in the same mixtures by the iiPCR machine, whereas no fluorescent signal was detected in lanes 1 to 6.

### 3. Real-time PCR

Real-time PCR assay were carried out in an ABI StepOnePlus™ real time PCR machine (Applied BioSystem, Life Technologies, CA, USA) using diluted pTA-ISAV plasmid (10¹, 10², 10³, 10⁴, 10⁵, 10⁶ copies). Real-time PCR assays were performed with 2 µl pTA-ISAV plasmid, 0.01-2 µM forward primer ISAVF1 (SEQ ID NO: 18), 0.01-2 µM reverse primer ISAVR1 (SEQ ID NO: 20), and 0.01-2 µM probe ISAVP2 (5' FAM- CGATGACTCTCTACTGTGTGA-MGB 3', SEQ ID NO: 38) in a total volume of 20 ul by using a commercial kit (such as, but not limited to, OneStep PrimeScript^{™} RT-PCR Kit; Takara Bio Inc., Japan). The program included an incubation period at 42°C 5min, 94°C for 10 sec, and 40 cycles of 94°C for 10 sec and 60°C for 30 min. Fluorescence measurements were recorded at the 60°C step.

As shown in FIG. 5F, the standard curve for the serial dilutions (10-fold) of the pTA-IPNV plasmid was calculated for the real-time PCR assays. At least 100 copies of pTA-ISAV plasmid can be detected. The R² value of the standard curve is 0.992, indicating the primers and the probe of the present invention can be used in real-time PCR to produce reliable outcomes.

The results also demonstrate that the primers and the probe can be used in iiPCR amplification to detect the existence of infectious salmon anemia virus (ISAV) with good sensitivity and specificity.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A method for detecting *Candidatus Branchiomonas cysticola* in coldwater fish, comprising:
providing a sample potentially containing one or more nucleotide sequences of a pathogen in a coldwater fish;
providing an oligonucleotide primer pair defining the 5' ends of two complementary strands of a double stranded target sequence on the one or more nucleotide sequences of the pathogen, the oligonucleotide primer pair comprising a first primer and a second primer;
providing an oligonucleotide probe having a sequence complementary to a segment of the double stranded target sequence, a fluorescer molecule attached to a first location on the oligonucleotide probe, and a quencher molecule attached to a second location on the oligonucleotide probe such that the quencher molecule substantially quenches the fluorescer molecule whenever the oligonucleotide probe is not hybridized to the segment of the double stranded target sequence and such that the fluorescer molecule is substantially unquenched whenever the oligonucleotide probe is hybridized to the segment of the double stranded target sequence;
providing a polymerase;
blending the sample, the oligonucleotide primer pair, the polymerase, deoxyadenosine triphosphates (dATPs), deoxycytidine triphosphates (dCTPs), deoxyguanosine triphosphates (dGTPs), and deoxythymidine triphosphates (dTTPs) in a container to form a polymerase chain reaction (PCR) mixture;
subjecting the PCR mixture to insulated isothermal polymerase chain reaction (iiPCR) by heating the bottom of the container at a fixed temperature to form a PCR product; and
detecting the PCR product to identify the double stranded target sequence;
wherein the first primer has the sequence of SEQ ID NO: 1, the second primer has the sequence of SEQ ID NO: 2 and the oligonucleotide probe has the sequence of SEQ ID NO: 3; or wherein the first primer has a sequence selected from then group consisting of SEQ ID NOs: 23, 25, and 27, the second primer has a sequence selected from the group consisting of SEQ ID NOs: 24, 26, and 28 and the oligonucleotide probe has the sequence of SEQ ID NO: 29.

2. A set of oligonucleotides for detecting *Candidatus Branchiomonas cysticola* in coldwater fish, comprising:
a first primer,
a second primer, and
an oligonucleotide probe having a sequence, a fluorescer molecule attached to a first location on the oligonucleotide probe, and a quencher molecule attached to a second location on the oligonucleotide probe;
wherein the first primer has the sequence of SEQ ID NO: 1, the second primer has the sequence of SEQ ID NO: 2 and the oligonucleotide probe has the sequence of SEQ ID NO: 3; or wherein the first primer has a sequence selected from the group consisting of SEQ ID NOs: 23, 25, and 27, the second primer has a sequence selected from the group consisting of SEQ ID NOs: 24, 26, and 28 and the oligonucleotide probe has then sequence of SEQ ID NO: 29.

## Patentansprüche

1. Verfahren zum Detektieren von Candidatus Branchomonas cysticola in Kaltwasserfischen, umfassend:
Bereitstellen einer Probe, welche potentiell eine oder mehrere Nukleotid-Sequenzen eines Pathogens in einem Kaltwasserfisch enthält;
Bereitstellen eines Oligonukleotid-Primerpaars, welches die 5'-Enden von zwei komplementären Strängen einer Doppelstrang-Zielsequenz auf dem einen oder auf den mehreren Nukleotid-Sequenzen des Pathogens hat, wobei das OligonukleotidPrimer-Paar einen ersten Primer und einen zweiten Primer umfasst;
Bereitstellen einer Oligonukleotid-Sonde mit einer Sequenz, welche zu einem Segment der Doppelstrang-Zielsequenz komplementär ist, einem Fluoreszenz-Molekül, welches an einem ersten Ort auf der Oligonukleotid-Sonde befestigt ist, und einem Löschmolekül, welches an einem zweiten Ort der Oligonukleotid-Sonde befestigt ist, so dass das Löschmolekül das Fluoreszenz-Molekül substantiell löscht, wenn die Oligonukleotid-Sonde nicht an dem Segment der Doppelstrang-Zielsequenz hybridisiert ist, und so dass das Fluoreszenz-Molekül substantiell nicht gelöscht ist, wenn die Oligonukleotid-Sonde an dem Segment der Doppelstrang-Zielsequenz hybridisiert ist;
Bereitstellen einer Polymerase;
Mischen der Probe, des Oligonukleotid-Primerpaars, der Polymerase, Deoxyadenosin-Triphosphate (dATPs), Deoxycytidin-Triphosphate (dCTPs), Deoxyguanosin-Triphosphate (dGTPs), und Deoxythymidin-Triphosphate (dTTPs), in einem Behälter, um eine Polymerase-Kettenreaktion (PCR) Mischung zu bilden;
Überführen der PCR-Mischung zu einer isolierten isothermalen Polymerasekettenreaktion (iiPCR) durch Heizen der Unterseite des Behälters auf eine fixierte Temperatur um ein PCR Produkt zu bilden; und
Bestimmen des PCR Produkts um die Doppelstrang-Zielsequenz zu identifizieren;
wobei der erste Primer die Sequenz der SEQ ID NO: 1 hat, der zweite Primer die Sequenz der SEQ ID NO: 2 hat, und die Oligonukleotid-Sonde die Sequenz der SEQ ID NO: 3 hat; oder
wobei der erste Primer eine Sequenz hat, welche ausgewählt ist, aus der Gruppe bestehend aus SEQ ID NOs: 23, 25 und 27, wobei der zweite Primer eine Sequenz hat, welche ausgewählt ist, aus der Gruppe bestehend aus SEQ ID NOs: 24, 26, und 28, und wobei die Oligonukleotid-Sonde die Sequenz der SEQ ID NO: 29 hat.

2. Kit aus Oligonukleotiden zum Detektieren von Candidatus Branchomonas cysticola in Kaltwasserfischen, umfassend:
einen ersten Primer,
einen zweiten Primer, und
eine Oligonukleotid-Sonde mit einer Sequenz, einem Fluoreszenz-Molekül, welches an einem ersten Ort an der Oligonukleotid-Sonde befestigt ist, und einem Löschmolekül, welches an einem zweiten Ort der Oligonukleotid-Sonde befestigt ist,
wobei der erste Primer die Sequenz der SEQ ID NO: 1 hat, wobei der zweite Primer die Sequenz der SEQ ID NO: 2 hat, und die Oligonukleotid-Sonde die Sequenz der SEQ ID NO: 3 hat; oder
wobei der erste Primer eine Sequenz hat, welche ausgewählt ist, aus der Gruppe bestehend aus SEQ ID NOs: 23, 25 und 27, wobei der zweite Primer eine Sequenz hat, welche ausgewählt ist, aus der Gruppe bestehend aus SEQ ID NOs: 24, 26, und 28, und wobei die Oligonukleotid-Sonde die Sequenz der SEQ ID NO: 29 hat.

## Revendications

1. Procédé pour détecter l'agent Candidatus Branchiomonas cysticola chez un poisson d'eau froide, comprenant :
la fourniture d'un échantillon qui contient potentiellement une ou plusieurs séquences(s) de nucléotides d'un pathogène chez un poisson d'eau froide ;
la fourniture d'une paire d'amorces oligonucléotidiques qui définit les extrémités 5' de deux brins complémentaires d'une séquence cible à deux brins sur les une ou plusieurs séquence(s) de nucléotides du pathogène, la paire d'amorces oligonucléotidiques comprenant une première amorce et une seconde amorce ;
la fourniture d'une sonde oligonucléotidique qui comporte une séquence qui est complémentaire par rapport à un segment de la séquence cible à deux brins, une molécule d'agent fluorescent qui est liée à un premier site sur la sonde oligonucléotidique et une molécule d'agent d'extinction qui est liée à un second site sur la sonde oligonucléotidique de telle sorte que la molécule d'agent d'extinction réalise de façon substantielle l'extinction de la molécule d'agent fluorescent chaque fois que la sonde oligonucléotidique n'est pas hybridée sur le segment de la séquence cible à deux brins et de telle sorte que la molécule d'agent fluorescent ne soit pas soumise à extinction de façon substantielle chaque fois que la sonde oligonucléotidique est hybridée sur le segment de la séquence cible à deux brins ;
la fourniture d'une polymérase ;
le mélange de l'échantillon, de la paire d'amorces oligonucléotidiques, de la polymérase, de triphosphates de désoxyadénosine (dATP), de triphosphates de désoxycytidine (dCTP), de triphosphates de désoxyguanosine (dGTP) et de triphosphates de désoxythymidine (dTTP) dans un récipient de manière à former une mixture de réaction de chaîne polymérase (PCR) ;
la soumission de la mixture PCR à une réaction de chaîne polymérase isotherme isolée (iiPCR) en chauffant le fond du récipient à une température fixe de manière à former un produit de PCR ; et
la détection du produit de PCR de manière à identifier la séquence cible à deux brins ; dans lequel :
la première amorce comporte la séquence de SEQ ID NO: 1, la seconde amorce comporte la séquence de SEQ ID NO: 2 et la sonde oligonucléotidique comporte la séquence de SEQ ID NO: 3 ; ou dans lequel :
la première amorce comporte une séquence qui est sélectionnée parmi le groupe qui est constitué par SEQ ID NO: 23, 25 et 27, la seconde amorce comporte une séquence qui est sélectionnée parmi le groupe qui est constitué par SEQ ID NO: 24, 26 et 28 et la sonde oligonucléotidique comporte la séquence de SEQ ID NO: 29.

2. Ensemble d'oligonucléotides pour détecter l'agent Candidatus Branchiomonas cysticola chez un poisson d'eau froide, comprenant :
une première amorce ;
une seconde amorce ; et
une sonde oligonucléotidique qui comporte une séquence, une molécule d'agent fluorescent qui est liée à un premier site sur la sonde oligonucléotidique et une molécule d'agent d'extinction qui est liée à un second site sur la sonde oligonucléotidique ; dans lequel :
la première amorce comporte la séquence de SEQ ID NO: 1, la seconde amorce comporte la séquence de SEQ ID NO: 2 et la sonde oligonucléotidique comporte la séquence de SEQ ID NO: 3 ; ou dans lequel :
la première amorce comporte une séquence qui est sélectionnée parmi le groupe qui est constitué par SEQ ID NO: 23, 25 et 27, la seconde amorce comporte une séquence qui est sélectionnée parmi le groupe qui est constitué par SEQ ID NO: 24, 26 et 28 et la sonde oligonucléotidique comporte la séquence de SEQ ID NO: 29.
